# EUROPÄISCHE PATENTSCHRIFT

(11) **EP 1 089 783 B1**
(45) Veröffentlichungstag und Bekanntmachung des Hinweises auf die Patenterteilung: **29.09.2004**
(21) Anmeldenummer: 99932708.3
(22) Anmeldetag: 22.06.1999
(51) Int. Cl.: A61M 15/00

(54) **VORRICHTUNG ZUM LEERSAUGEN PULVERENTHALTENDER KAVITÄTEN**
DEVICE FOR EMPTYING CAVITIES CONTAINING POWDER BY MEANS OF SUCTION
DISPOSITIF POUR LE VIDAGE PAR ASPIRATION DE CAVITES CONTENANT DE LA POUDRE

(30) Priorität: 22.06.1998 DE 19827731; 09.07.1998 DE 19830713; 29.08.1998 DE 19839516; 04.12.1998 DE 19855851
(43) Veröffentlichungstag der Anmeldung: 11.04.2001
(73) Patentinhaber: AstraZeneca AB, 151 85 Södertälje (SE)
(72) Erfinder: VON SCHUCKMANN, Alfred, 47627 Kevelaer (DE)
(86) Internationale Anmeldenummer: PCT/EP1999/004304
(87) Internationale Veröffentlichungsnummer: WO 1999/066974

(56) Entgegenhaltungen:
- EP-A- 0 129 985
- EP-A- 0 467 172
- DE-A- 19 619 536
- US-A- 2 549 303
- US-A- 5 415 162

## Beschreibung

Die Erfindung betrifft eine Vorrichtung zum Leersaugen pulverenthaltender Kavitäten gemäß Gattungsbegriff des Anspruches 1 .

Bei den vorbekannten Lösungen dieser Art (US-PS 25 49 303) ist die Kavität in einem massiven Unterteil vorgesehen, welches eine topfförmige Vertiefung besitzt auf dessen Topfrand die Deckfolie gespannt ist. Das Saugrohr ist an seinem vorderen Ende mit einem Ringkragen ausgestattet, der deckelartig auf die äußere Topfmantelwand aufschiebbar ist. Zurückspringend gegenüber diesem Rand ist im Oberteil ein Kegelstumpf vorgesehen, dessen vordere schmälere Kegelstumpfstirnfläche beim Niederdrücken des Saugrohres die Deckfolie durchstößt. In der Innenfläche der umfassenden Wand des Ringkragens ist ein kanalförmiger Querschnitt belassen zum Lufteintritt während des Leersaugens der Kavität. Die Lösungen sind bautechnisch und handhabungstechnisch unzureichend: Das äußere Umfassen unter Belassung eines nur kleinen Lufteintrittsquerschnittes macht es unmöglich, stets ein gleichmäßiges, insbesondere vollständiges Entleeren der Kavität zu erzielen. Dazu trägt auch bei, daß die vordere querschnittskleinere Kegelstumpfstirnfläche keine gezielte Öffnungstrennung der Deckfolie ermöglicht, so daß Fälle auftreten, wo unglücklich eingeklappte Abschnitte der Deckfolie Reste des auszusaugenden Pulvers in eine Verstecklage bringen, die auch noch dadurch begünstigt wird, daß sich möglicherweise solche Deckfolienabschnitte an die Kegelstumpfmantelfläche ansaugen. Auch die Tatsache, daß das Saugrohr in Relation zum Unterteil drehbar ist und u.U. sogar nach dem Durchstechen der Deckfolie bei der Handhabung noch gedreht wird, beeinträchtigt die Reproduzierbarkeit des Leersaugvorganges. Das macht den Einsatz im Bereich genau zu dosierender Medikamente unmöglich. Darüber hinaus ist das Unterteil, welches die napfförmige Kavität bildet, als Einwegteil gedacht und wird nach Durchstoßen der Deckfolie weggeschmissen, was einen erheblichen Teuerungsfaktor darstellt, abgesehen von der Lagerhaltung für die Fälle einer regelmäßigen medikamentösen Einnahme des Pulvers.

Eine andere, den letztgenannten Nachteil vermeidende Lösung ist vorbekannt durch die DE-OS 196 19 536. Dort ist ein Träger vorgesehen, dem eine übliche Blisterpackung zugeordnet werden kann mit einer Vielzahl von Kavitäten, die jeweils das Pulver in entsprechender Dosierung beinhalten. Man hat nicht nur den Vorteil der Wiederverwendbarkeit des Träges durch Auffüllen mit einer neuen Blisterpackung, sondern auch den Vorteil, daß stets mehrere Kavitäten bereitgestellt sind und der Patient bei medikamentöser Anwendung auch einen gewissen Kontrollcharakter hat hinsichtlich der bereits durchgestoßenen Deckfolien und der noch unbenutzten Kavitäten. Der Träger hat dabei eine Deckplatte, die einzelne mit den Kavitäten des Blisters fluchtende Löcher besitzt. In diesen wird das Saugrohr zunächst positioniert und dann durch weiteres Einstecken bis in die Saugstellung gebracht. Dem Saugrohr sind dabei am vorderen Ende schneidende Flanken zugeordnet, die eine bestimmte, das Leersaugen optimierende Durchtrennung der jeweiligen Deckfolie der Kavität gewährleisten. Die notwendigen Lufteintrittsquerschnitte, die das Nachströmen von Luft in die Kavitäten beim Leersaugen derselben gestatten, sind dabei als axiale Rohrkanäle des Saugrohres gestaltet, deren vordere Mündungsquerschnitte nach Durchstoßen der Deckfolie in der Kavität freiliegen.

Die diesbezüglichen Lösungen sind nachteilig im Hinblick darauf, daß die frei vorstehenden Messer recht empfindlich sind gegen Beschädigungen beim ersten Positionieren des Saugrohes in dem Loch des Trägers. Um sie überhaupt beim Durchstechen der Deckfolie einigermaßen gegen Beschädigung zu schützen, muß das Saugrohr in den Löchern bereits längsgeführt sein, bevor das Messer auf die Deckfolie aufsetzt. Das bedingt eine relativ große Achslänge dieser Einstecklöcher des Trägers, was wiederum den Nachteil hat, daß die Blisterdeckfolie recht weit unterhalb der Oberseite des Trägers liegt und es dadurch oft nur schwer kontrollierbar ist, ob eine bestimmte Kavität schon leergesaugt ist oder nicht. Macht man bei dieser vorbekannten Lösung das Einsteckende des Saugrohres unrund, so ist zwar ein Drehen des Saugrohres nach ineinandergreifen dieses unrunden Bereiches in das entsprechend unrunde Loch des Trägers und die damit verbundene Beschädigung des Messers durch Drehen desselben in der geschnittenen Deckfolie verhindert; die Lösung hinsichtlich der Lufteintrittsquerschnitte diese als Rohrkanäle des Saugrohres auszubilden wird dadurch jedoch noch komplizierter. Ein möglichst gleichmäßiges Einsaugen dieser Luft von allen Seiten in die Kavität, was von Hause aus erst alle Kavitätsbereiche möglichst gleichstellt, ist nicht erreichbar.

Aufgabe der Erfindung ist es, eine gattungsgemäße Vorrichtung so auszubilden, daß sie preisgünstiger herstellbar und sicherer zu handhaben ist.

Diese Aufgabe wird beim Anspruch 1 dadurch gelöst, daß das Saugrohr in vom Eintrittsloch für die Messer getrennten Schlitzen des Trägers positioniert ist

Hierdurch liegt auch der Vorteil einer konzentrierten Beherrschung der Vorrichtung überhaupt vor. Die mit den Löchern ausgestattete Decke des Trägers, unter welcher der Blister liegt, kann recht dünn sein; trotzdem liegt eine rechtzeitige, genügende Positionierung des Saugrohres vor, rechtzeitig bevor die Messer in das Loch einfahren. Die Blister-Deckfolie kann dadurch wegen der kürzeren Achslänge der Löcher besser sichtbar liegen, was die sichere Handhabung insbesondere bei älteren Personen erleichtert. Bei der Version mit den flachen Fortsätzen, die in die Schlitze eintauchen, ist das Saugrohr ab dem ersten Einstecken der Fortsätze drehgesichert. Die geschützt zurückversetzt liegenden Schneidflanken können auch in Relation zum Loch des Trägers so gestaltet sein, daß ein optimaler Lufteintrittsquerschnitt zur Kavität frei bleibt, weil dieser Schneidenbereich des Saugrohres mit einer Positionierung und Führung desselben nichts mehr zu tun hat. Das fördert nicht nur durch die frei von allen Seiten einströmende Luft die stets gleichmäßige Entleerung der Kavität, sondern vermeidet auch, daß sich nach vielfachem Gebrauch des Saugrohres in diesem Reste des Pulvers ansammeln, wie es z.B. in den Lufteinlaßkanälen der vorbekannten Lösung gemäß OS 196 19 536 vorkommen kann. Bezüglich der Längs-Führung bzw. Positionierung des Saugrohres erweist es sich als günstig, daß das Saugrohr in beiderseits der Löcher liegenden Schlitzen des Trägers führbar ist. Es liegt dann eine Art schlittenartiger Verschiebbarkeit vor.

Wenn die durchstoßenden Flanken als flache, überragende Fortsätze gestaltet sind, so bilden diese einen ausgezeichneten Schutz für die Schneide und etwaige Stößelblätter. Sie stellen überdies sicher, daß große Lufteintrittsquerschnitte verbleiben. Hierüber eröffnen sich weiterbildende Möglichkeiten im Hinblick auf die lösbare Zuordnung des Saugrohres zum Träger hin und dessen handhabungsgerechte Zuordnung auch bei Nichtgebrauch. Die Laschen, welche als Führungslaschen beim Positionieren und Einstecken des Saugrohres in die Löcher dienen, ermöglichen auch eine vorteilhafte Ausgestaltung durch schwenkbewegliche Zuordnung der Laschen zu dem Blisterpackungs-Träger. Das läßt sich auch für eine geführte Beweglichkeit nutzen, z.B. erst in in eine Stellung deckend über den Löchern, um dann einzufahren in unterhalb der Schlitze liegende Saugrohr-Positionierungsfreiräume. Die entsprechende Kulisse ist so gelegt, daß auf der Zwischenstrecke zur jeweiligen nächsten sauggerechten Position keine Berührung zwischen den durchstoßenden Flanken des Saugrohres und Partien des Trägers stattfindet. Die stech- bzw. lochungsaktive Flanke der Schneide wird so stets geschützt. Die Positionierungsfreiräume sind günstigerweise als von den Schlitzen ausgehende Stichkanäle ausgebildet. Die Führungslaschen können je einen Nocken aufweisen, die in die Stichkanäle einfahrbar sind. Die Endstellung dieser Einfahrbewegung ist die Durchstoßendstellung. Weiter ist vorgesehen, daß das Saugrohr einen in die Löcher einfahrenden Stößelabschnitt besitzt, der - zurückversetzt zu den durchstoßenden Flanken - derart angeordnet ist, daß er sich zu einer Linearführung des Saugrohres ergänzt, wenn die Nocken in die Stichkänale eintreten. In diesem Zusammenhang erweist es sich noch als vorteilhaft, daß dem Stößelabschnitt benachbarte Flächen des Saugrohres in Durchstoßstellung auf die Bereiche seitlich der Löcher des Trägers aufsetzen. Das ergibt eine Unterstützung der verkippungsfreien Durchstoßstellung. Weiter ist die Vorrichtung gekennzeichnet durch einen das abklappbare Saugrohr überfangenden Deckel des Trägers.

Eine Fesselung des Saugrohres am Blister oder Träger hat den Vorteil, daß das Saugrohr nicht zu lange benutzt wird. Über Schlitze in der Blisterpackung, in welche Schlitze die Fortsätze des Saugrohres eintauchen/durchtauchen müssen, kann sogar der Blister auf die Benutzung individualisiert sein.

Es läßt sich ein gedrungener Aufbau der Vorrichtung erreichen. Vorteilhaft ist die Führung zwischen Saugrohr und Träger derart, daß das Saugrohr nacheinander in zwei Ebenen beweglich ist. Diese Gliederung sieht eine zweifache Positionierung, und zwar quer zu den Löchern und in einer Stechebene vor. Eine raumsparende Nichtgebrauchsstellung für das Saugrohr wird dabei erreicht, wenn in einer der beiden Ebenen das Saugrohr sowohl klappbar als linear geführt verschieblich ist.

Blisterpackungsseitig ermöglicht sich dabei eine ebenfalls vorteilhafte Ausgestaltung: Sie besteht z.B. darin, daß die Blisterpackung zu einem Stab mehrseitigen Querschnitts mit innenliegenden Kavitäten gefaltet und in mehreren um seine Längsachse umgewendeten Stellungen in den Träger einschiebbar ist. Eine solche Stabform ist raumsparend. Zur Unterbringung der Kavitäten wird der bei Faltung ohnehin entstehende Innenraum genutzt. Das kann bis zu einer berührenden Anlage der Kavitäten Rücken an Rücken gehen, was bei kongruenter Ausrichtung zu einem gegenseitigen Abstützen führt. Günstig ist es dabei, wenn der Blisterpackungs-Stab in Längsrichtung in den Träger einschiebbar ist. Damit liegt eine konsequente Nutzung des Gedankens der Längsführung vor. Eine räumlich vorteilhafte Querschnittsgestalt des Blisterpackungs-Stabes besteht darin, daß er quadratischen Querschnitt aufweist und auf allen vier Breitseiten je eine Kavitäten-Reihe hat. Obwohl der Blisterpackungs-Stab auch allein aus der Faltung schon eine gewisse Steifigkeit besitzt bzw. Formtreue aufbringt, können diese Eigenschaften noch erhöht werden, indem der Blisterpackungs-Hohlstab von einem Einschubkäfig umfaßt ist, der an allen Seitenwänden mit der Position der Kavitäten übereinstimmende Öffnungen ausbildet. Ihm wird dann der Träger zugeordnet. Es kann sich pro Breitseite um sieben Kavitäten handeln, beispielsweise als Wochenbedarf an Medikamentendosen. Wird auf eine berührende Anlage der Rücken der Wände der Kavitäten verzichtet, kann eine vorteilhafte Maßnahme darin bestehen, daß der Träger einen in den Innenquerschnitt des Blisterpackungs-Hohlstabes eintauchenden Kernstab aufweist, dessen mindestens dem Saugrohr zugekehrte Flanke als Stützboden für die Kavitätenunterseite gestaltet ist. Bevorzugt ist jedoch der Kernstab so gestaltet, daß alle vier Flanken des Kernstabes als Stützboden ausgebildet sind. Das erbringt zugleich eine Innenführung. Um zu vermeiden, daß der Blisterpackungs-Hohlstab bspw. beim Laden/Neuladen des Trägers aus dem ihn schützend umgebenden Einschubkäfig herausrutscht, ist vorgesehen, daß der Blisterpackungs-Hohlstab in Einschubendstellung im Käfig verrastet. Es kann sich um einfache Rastwarzen handeln, die in korrespondierende Vertiefungen einschnäppern. Vorteilhaft ist es dabei weiter, wenn die (geschlossene) Stirnseite des Einschubkäfigs mit Benutzungssymbolen ausgestattet ist. Unter Beibehaltung des Prinzips der Längenerstreckung bzw. Stabform ist auch der Träger entsprechend ausgebildet. Das besteht lösungsmäßig darin, daß der Träger stabförmig gestaltet ist und an einer Seitenfläche eine geradlinige Reihe von Saugrohr-Durchtritts-Löchern aufweist, welche Löcher deckend liegen zu den Blisterpackungs-Kavitäten. Die Löcher sind durch schmälere, freie Querschnitte miteinander verbunden. Anstelle eines trägerseitigen Kernstabes kann die entsprechende Steifigkeit der prismatischen Blisterpackung auch von dieser selbst ausgehen. Das ist erreicht durch einen die Blisterpakkungs-Kavitäten formenden - massiven - Stab als Kernstück, dessen Seitenflächen die Blisterpackung-Deckfolie aufweisen. So liegt ein Massivmagazin vor. Das läßt sich bevorzugt aus Glas herstellen, um diesbezüglichen Aufbewahrungsforderungen hinsichtlich bestimmter Medikamentenpulver gerecht zu werden (adäquat der Aufbewahrung in Glas-Verpackungen). Denkbar sind auch andere Materialien wie Keramik und fester Kunststoff. Um auch hier den Wendevorteil zu bekommen, sind die oben beschriebenen Verhältnisse zugrundegelegt. Dabei ist es auch so, daß die Kavitäten aller Seitenflächen jeweils auf einer gemeinsamen Querebene des Kernstückes liegen. Der Stab kann dann auch die Führungsmittel für das Saugrohr verkörpern, so daß auch so wiederum Saugrohr und Stab eine zusammenhängende Einheit darstellen. Das hat den Vorteil, daß bei Erneuerung des (leeren) Blisters auch das Saugrohr erneuert wird, was vermeidet, daß ein und dasselbe Saugrohr zu lange benutzt wird und so beispielsweise durch Rückstände oder dergleichen medizinisch unsicher wird. Eine Form der zusammenhängenden Einheit besteht dabei darin, daß das Saugrohr "huckepack" auf dem Stab oder Käfig sitzt vorzugsweise in einer Zapfen/Loch-Steckverbindung und beim Einschieben des Stabes in den Träger an die Führung/Führungsnuten des Trägers übergeben wird am besten unter selbständigem Lösen der Zapfen/Loch-Steckverbindung. Das Saugrohr bleibt dabei am Träger und ist klappbar in die Einsteck-Bereitschaftsstellung auch wenn man den Stab aus dem Träger herauszieht, z.B., um ihn nach Drehen wieder einzuschieben. Soll ein neuer Stab - mit gefüllten Kavitäten - in Gebrauch genommen werden, kann man diesen erst in den Träger einschieben, wenn das Saugrohr des voraufgegangenen Stabes aus den Führungen des Trägers herausgezogen ist, um ihn dann wegzuwerfen. Anspruchsmäßig ist das im einzelnen dadurch gekennzeichnet, daß das Saugrohr in lösbarer Verbindung am Stab oder Käfig sitzt und sich die Verbindung beim Einschieben des Stabes oder Käfigs in den Träger selbsttätig löst unter Übergabe des Saugrohres an eine Führung des Trägers. Dabei erweist es sich als vorteilhaft, daß eine Flachseite des Saugrohres Zapfen aufweist, die reibschlüssig in Löchern des Stabes, Kernstücks oder Käfigs eintreten. Die Reibungskräfte sind so eingestellt, daß das Saugrohr nur willensbetont aus seiner Reiterstellung bewegt werden kann. Konkret besteht eine vorteilhafte Maßnahme darin, daß das Lösen des Saugrohres bei der Übergabe desselben an die Führung des Trägers durch Schrägen einlaufseitiger Hubnocken einer Zahnreihe bewirkt wird. Deren Bedeutung wird weiter unten erläutert. Im Interesse einer raumsparenden Unterbringung des Saugrohres unter Durchlaufen der Kavitätenreihe ist es dabei vorteilhaft, daß das Saugrohr zufolge gelenkiger Anordnung an seinen Führungslaschen um eine Querachse umwendbar ist. Die entsprechende Gelenkigkeit sichert ein gegengerichtetes Umklappen des Saugrohres. In diesem Zusammenhang erweist es sich dabei als vorteilhaft, daß die paarig angeordneten Laschen wendesymmetrisch gestaltet sind und sich in Abklappstellung des Saugrohres an den Schultern desselben abstützen. Saugrohr und Laschen bilden eine starre Einheit. Eine benutzungstechnisch vorteilhafte Ausgestaltung ergibt sich dadurch, daß die Stirnwand des Käfigs mit dem Winkel der Umwendbarkeit des Käfigs entsprechend positionierten Originalitätsbrücken ausgestattet ist, von denen die dem Deckel jeweils zugekehrt liegende Originalitätsbrücke durch eine Nase des Dekkels bei der Schließbewegung desselben zerstörbar ist. Der Benutzer kann so ablesen, welche Kavitäten-Reihe noch gefüllt ist. Eine solche Originalitätsbrücke kann farblich abgehoben sein, so daß ihr Fehlen besonders augenfällig ist. Eine weitere Maßnahme zur Erzielung einer übersichtlicheren Handhabung der Vorrichtung verkörpert sich dadurch, daß der kappenförmige Deckel im Wege der Klapp-/Schiebebewegung in eine jenseits des rückwärtigen Gehäuseendes liegende Absenkstellung bewegbar ist. Der Deckel verschwindet so aus dem Entnahmebereich und verstellt auch nicht die Sicht. Die technischen Mittel sehen dabei so aus, daß der Deckel einen quer abstehenden Ausleger besitzt, der gelenkig und verschiebbar längs der oberen Gehäuseseite geführt ist und zusätzlich über einen Lenker mit der unteren Gehäuseseite in Verbindung steht. Dabei ist es weiter so, daß ein deckelseitiger Anlenkpunkt des Lenkers in einem Längsschlitz des Deckels verschieblich ist. Dabei bringt die Erfindung in Vorschlag, daß der Ausleger paarig gestaltet ist und jeder Ausleger mit einem Führungszapfen in einen Führungsschlitz des Gehäuses zum vorderseitigem Ende des Gehäuses verschiebbar ist. Die Silhouette des abgeklappten Saugrohres berücksichtigend, wird weiter so vorgegangen, daß der Führungsschlitz sich am rückwärtigen Gehäuseende in einen abwärtsgerichteten Abschnitt fortsetzt derart, daß dieser Abschnitt beim Öffnen des Deckels jeweils eine Hub- bzw. Senkbewegung des Deckels erzwingt derart, daß dessen Deckelrandkante das Saugrohr berührungslos überfährt. Die Bedeutung der Zahnreihe respektive Zahnlükken aufgreifend, ist eine vorteilhafte Weiterbildung gekennzeichnet durch eine kammartig vorstehende Zahnreihe an jeder Seitenwand des Gehäuses, in deren Lücken sich das Saugrohr bei der Durchstechbewegung des Saugrohres führt. Günstig ist es schließlich, daß das Saugrohr in der niedergesteckten Stellung verrastet ist.

Weiterhin kann der die Blisterpackung aufnehmende Träger sich in eine Halterung fortsetzen, in welche die ausrastbaren Achsstummel eintreten, insgesamt mit der Blisterpackung in ein deckelverschlossenes Gehäuse einbringbar ist derart, daß am Saugrohr verrastende Mitnehmer das Saugrohr um die Achsstummel schwenkend in eine griffbereite Lage hochklappen. Das Saugrohr steht so greifgerecht. Die Achsstummel lassen sich schnäpperartig überwinden. Bezüglich des Gehäuses handelt es sich dabei zweckmäßig um eine Klappschachtel. Dessen Deckelinnenseite können die genannten Mitnehmer angeformt sein. Längsrippen am Boden des Unterteiles der Klappschachtel treten beim Einlegen/Einschieben des Trägers (plus daran befestigtem Blister) zwischen je zwei Kavitätenreihen. Im Scheitel der Längsrippen sind deckend zu den Einzelschlitzen des Trägers und Blisters Einstecköffnungen für die freien Enden der Laschen vorgesehen.

Sodann bringt die Erfindung in Vorschlag, daß die Blisterpackungs-Deckfolie deckend zu den Einzelschlitzen im Träger und seitlich benachbart zu den Kavitäten Einsteckschlitze für Fortsätze besitzen.

Der Gegenstand der Erfindung ist nachstehend anhand mehrerer zeichnerisch veranschaulichter Ausführungsbeispiele näher erläutert. Es zeigt:

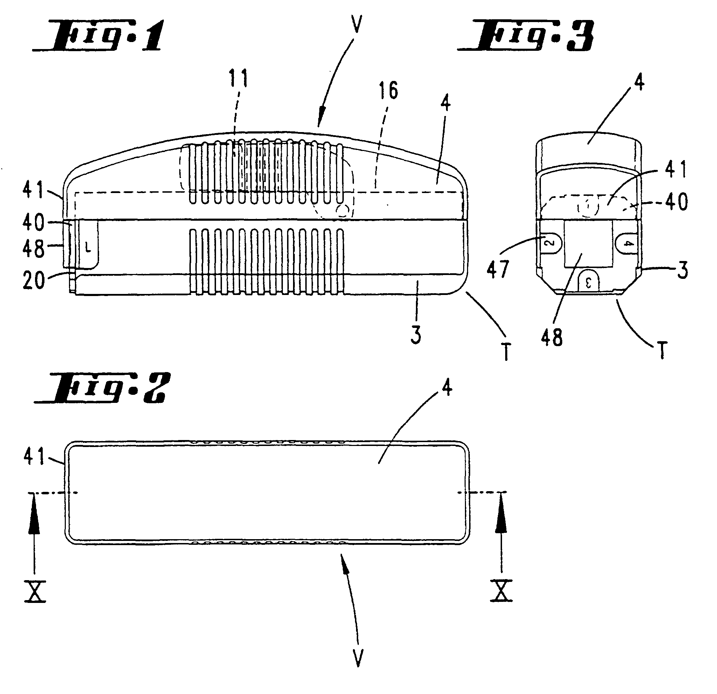

- Fig. 1: die erfindungsgemäße Vorrichtung in Seitenansicht, und zwar in etwa natürlicher Größe, gemäß dem ersten Ausführungsbeispiel,
- Fig. 2: die Draufsicht hierzu,

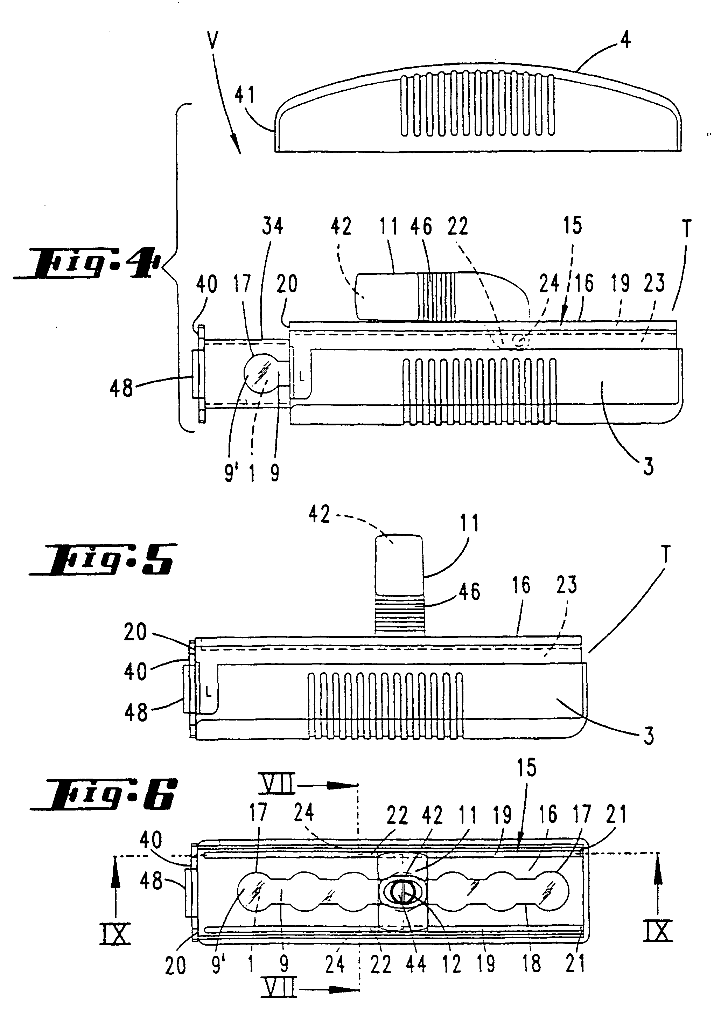

- Fig. 3: eine ladeseitige Stirnansicht der Vorrichtung,
- Fig. 4: die Vorrichtung in Seitenansicht bei geöffnetem Deckel und noch nicht vollständig zugeordnetem Einschubkäfig, enthaltend einen Blisterpackungs-Hohlstab und bei abgeklapptem Saugrohr,

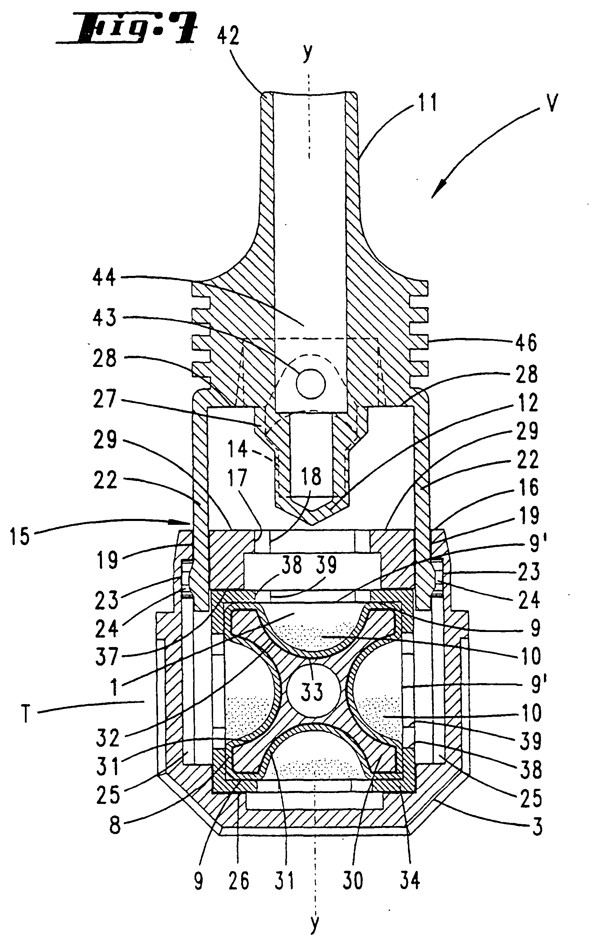

- Fig. 5: eine Darstellung wie Fig. 4, jedoch bei in Durchstoßstellung befindlichem, also aufgerichteten Saugrohr, benutzungsgerecht,
- Fig. 6: die Draufsicht auf Fig. 5,
- Fig. 7: den Schnitt gemäß Linie VII-VII in Fig. 6, vergrößert, bei in Verfahrstellung befindlichem Saugrohr,
- Fig. 8: eine der Fig. 7 entsprechende Darstellung, nun die Durchstoßstellung zeigend,
- Fig. 9: den Schnitt gemäß Linie IX-IX in Fig. 6, vergrößert, eine Bewegungsstudie des kippschlittenartig zugeordneten Saugrohres veranschaulichend,
- Fig. 10: den Schnitt gemäß Linie X-X in Fig. 2, vergrößert,

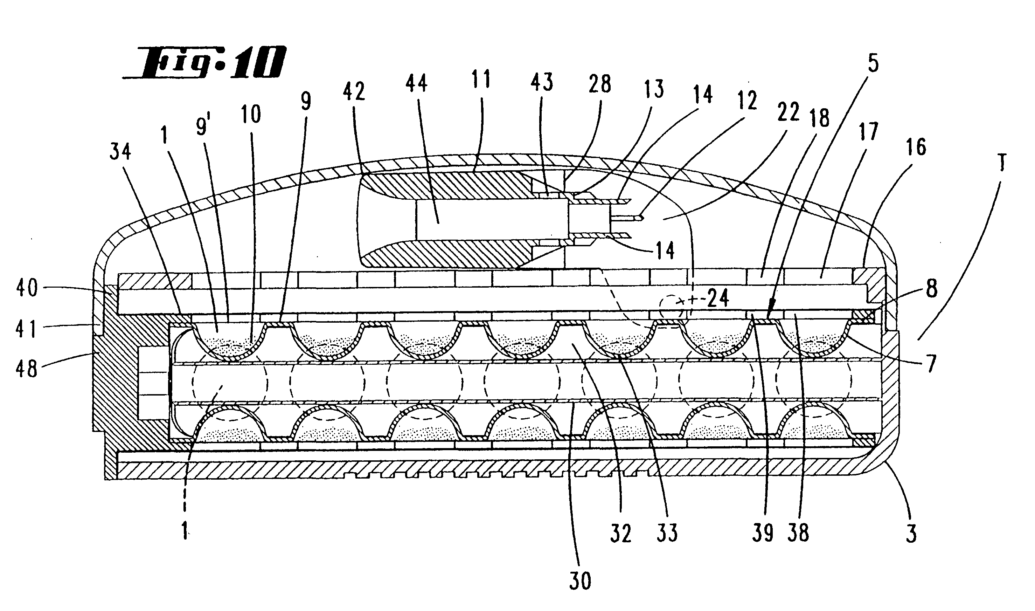

- Fig. 11: eine Schnittdarstellung wie Fig. 10, jedoch bei abgenommenem Deckel und in Durchstoßstellung befindlichem Saugrohr,
- Fig. 12: die Draufsicht auf eine Blisterpackung mit die Kavitäten verschließender Deckfolie,
- Fig. 13: die Blisterpackung zu einen Hohlstab verfaltet,
- Fig. 14: eine das Faltprofil verdeutlichende Stirnansicht des Blisterpackungs-Hohlstabes,
- Fig. 15: einen den Blisterpackungs-Hohlstab aufnehmenden Einschubkäfig in Seitenansicht,

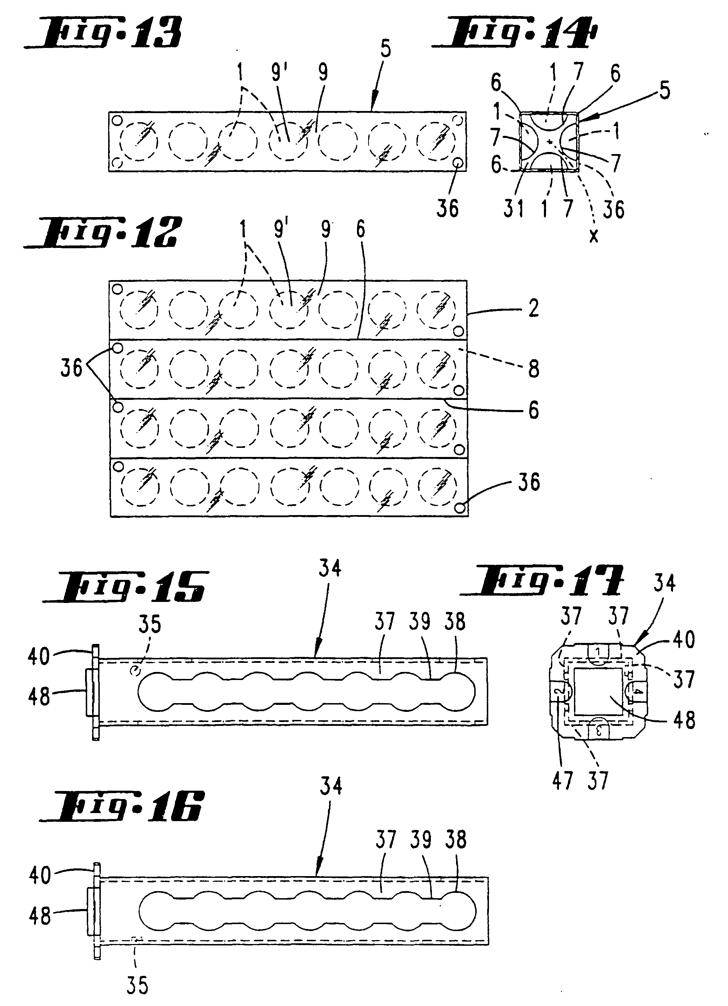

- Fig. 16: die Draufsicht hierzu,
- Fig. 17: eine der Fig. 3 entsprechende Stirnansicht, hier des Einschubkäfigs allein,
- Fig. 18: einen die Blisterpackungs-Kavitäten formenden Stab als Kernstück in Seitenansicht,
- Fig. 19: die Draufsicht hierzu,
- Fig. 20: eine Stirnansicht wie Fig. 17 und
- Fig. 21: den Schnitt gemäß Linie XXI-XXI in Fig. 18, vergrößert,
- Fig. 22: eine Seitenansicht einer abgewandelten Ausgestaltung als zweites Ausführungsbeispiel,
- Fig. 23: eine Weiterbildung der Vorrichtung gemäß dem zweiten Ausführungsbeispiel, und zwar in Seitenansicht, deckelgeschlossen,
- Fig. 24: die Draufsicht hierzu,

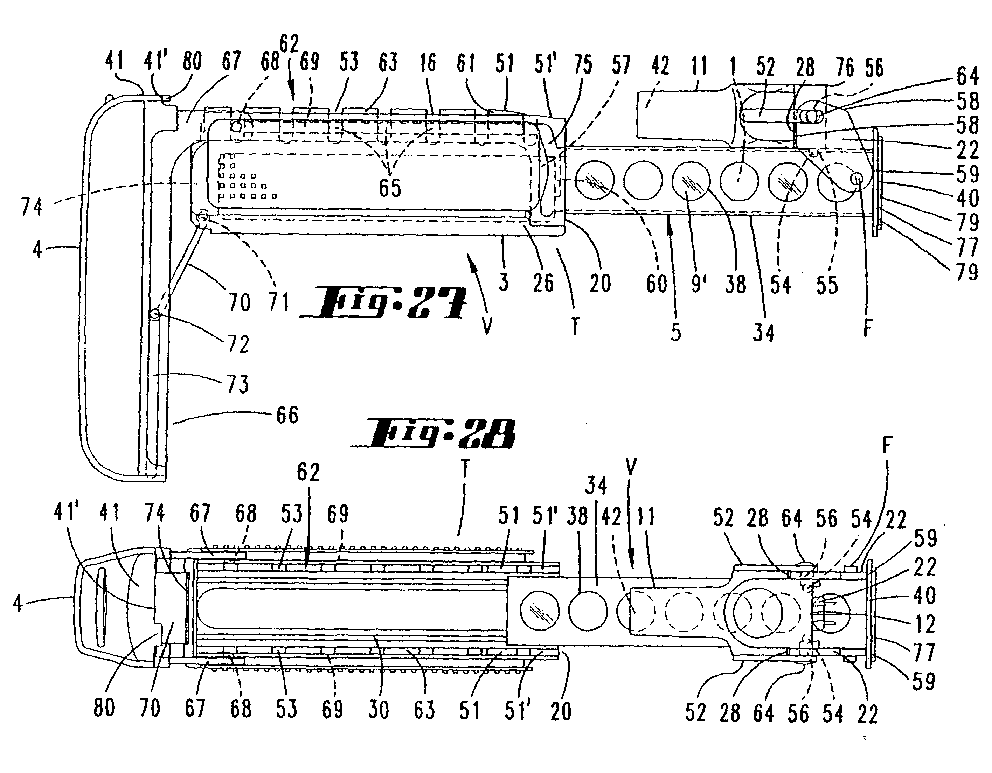

- Fig. 25: eine ladeseitige Stirnansicht der Vorrichtung,
- Fig. 26: die andere Stirnansicht der Vorrichtung,
- Fig. 27: die Vorrichtung in einer Beladestellung unter Zuordnen des das Saugrohr rittlings tragenden Käfigs zum Gehäuse bei dabei in eine Absenkstellung gebrachtem Deckel,
- Fig. 28: die Draufsicht hierzu,
- Fig. 29: Käfig mit Saugrohr in einer Aussteuerphase,
- Fig. 30: eine Darstellung wie Fig. 29, jedoch nach Aufheben der Verbindung zwischen Saugrohr und Käfig, respektive Stab,
- Fig. 31: die Vorrichtung bei in Durchstechstellung gebrachtem Saugrohr (mit in strichpunktierter Linienart eingetragener stechgerechter Hubstellung),
- Fig. 32: den Schnitt gemäß Linie XXXII-XXXII in Fig. 31, vergrößert,

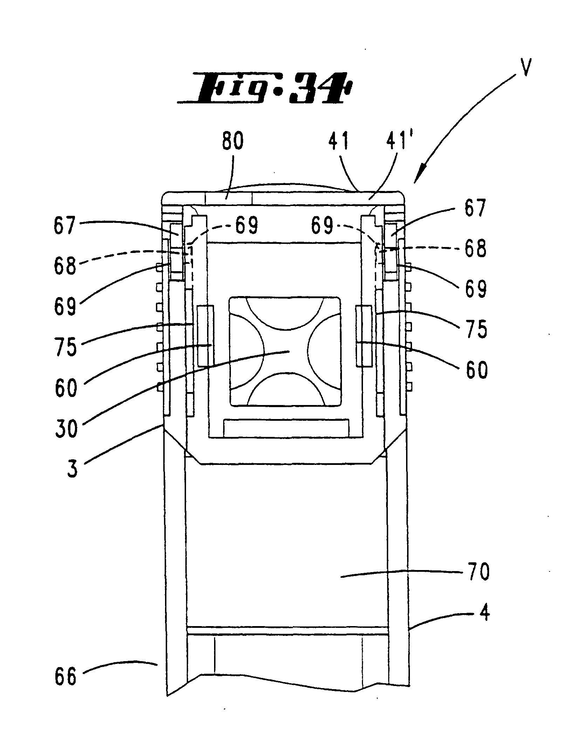

- Fig. 33: den Schnitt gemäß Linie XXXIII-XXXIII in Fig. 31 bei nun in ausgezogener Linienart wiedergegebenem Saugrohr, gleichfalls vergrößert,
- Fig. 34: die ladeseitige Stirnansicht der Vorrichtung, geöffnet,
- Fig. 35: Saugrohr und Käfig in andockgerechter Stellung, schaubildlich,

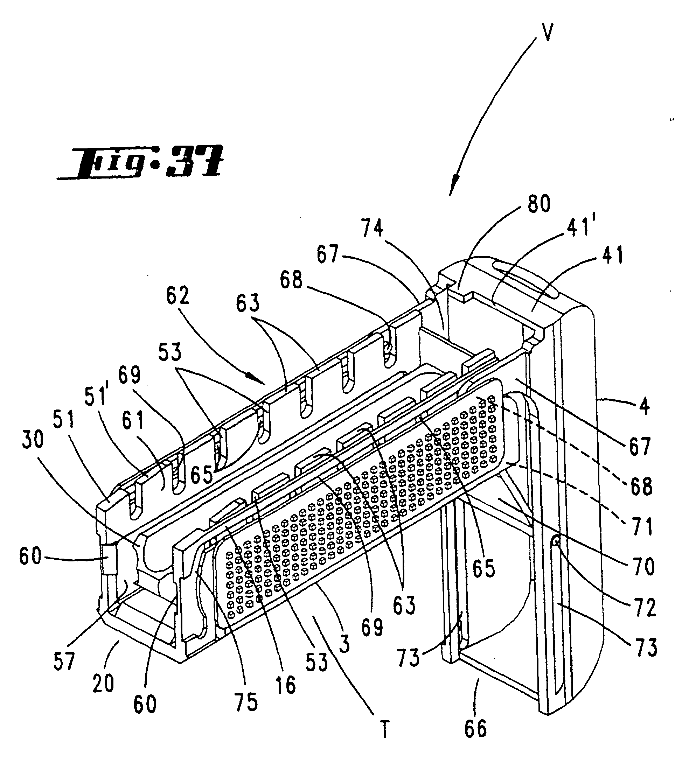

- Fig. 36: ein Schnittdetail im Bereich einer der Originalitätsbrücken,

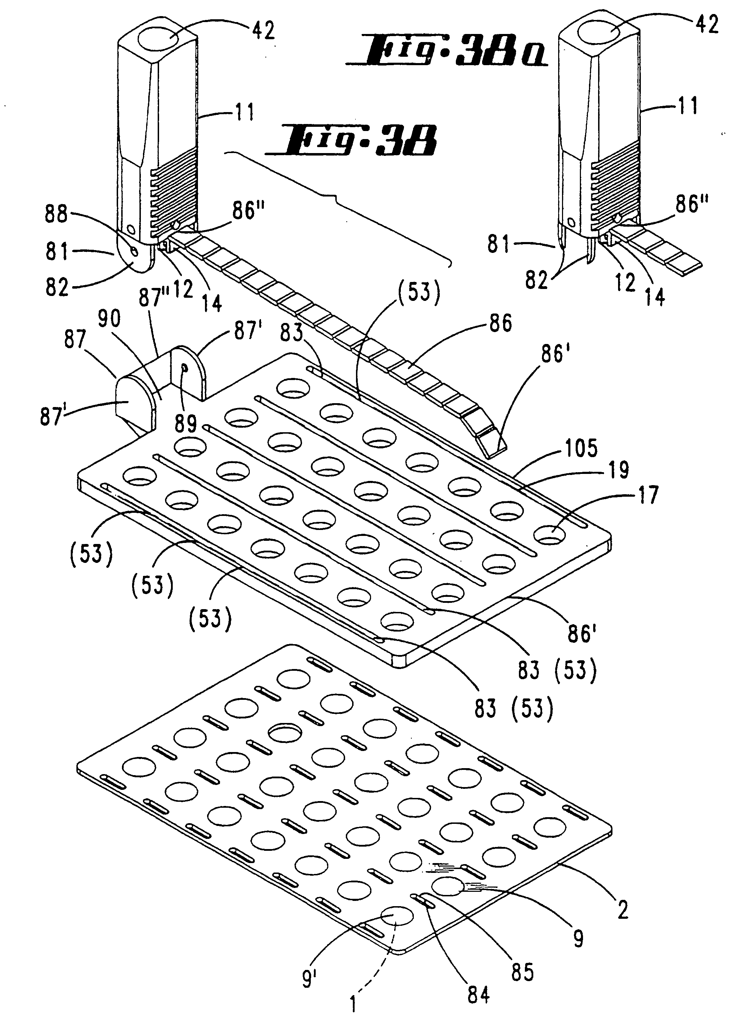

- Fig. 37: die ladegerechte Vorrichtung in Perspektive,
- Fig. 38: ein drittes Ausführungsbeispiel, wobei Fig. 38a die Fortsätze/Laschen 82, reduziert auf die Ecken eines Viereckes, zeigt,
- Fig. 39: einen vergrößerten Schnitt bei eingestecktem Saugrohr,
- Fig. 40: einen Querschnitt hierzu gemäß Linie XLI-XLI in Fig. 39,
- Fig. 41: diese Vorrichtung bei abgeklapptem Saugrohr, wieder in schaubildlicher Darstellung,
- Fig. 42: eine der Figur 41 entsprechende Darstellung, und zwar bei in einer Zwischenklappstellung befindlichem Saugrohr,
- Fig. 43: eine der Figur 41 entsprechende Darstellung, bei in eine Greifposition gebrachtem Saugrohr, veranlaßt durch Mitschlepporgane eines umgebenden, aufklappbaren Gehäuses,
- Fig. 43a: eine perspektivische Darstellung eines solchen aufklappbaren Gehäuses,
- Fig. 43b: einen Querschnitt durch einen Teilbereich des Gehäuses bei einliegendem Träger plus Blister und eingestecktem Saugrohr,
- Fig. 44: die Draufsicht auf Figur 43,

Die dargestelle Vorrichtung V zum Leersaugen pulverenthaltender Kavitäten 1 von Blisterpackungen 2 weist ein Gehäuse 3 auf. Letzteres ist deckelverschließbar. Der Deckel trägt beim ersten Ausführungsbeispiel das Bezugszeichen 4.

Bei der ersten und zweiten Ausführungsform bildet das Gehäuse 3 einen länglichen Träger T. Der ist stabförmig gestaltet und längsgehöhlt. Die Stablänge entspricht etwa dem Drei- bis Sechsfachen der Stabbreite. Bevorzugt liegt zumindest höhlungsmäßig diesbezüglich ein quadratischer Querschnitt vor.

Der Träger T ist von seinem einen Stirnende her bestückungszugänglich. Das andere Stirnende ist geschlossen.

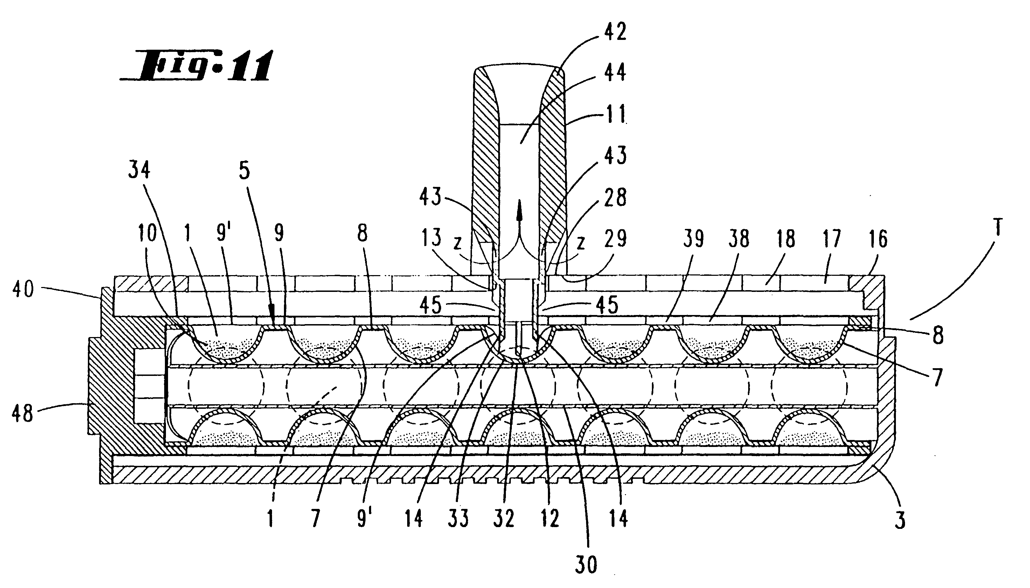

Das Bestückungsgut ist die in ihrem Querschnitt höhlungsmäßig angepaßte Blisterpackung 2. Diese weist hier dementsprechend Stabform auf (es sei auf die Figuren 13 und 14 verwiesen).

Ausgehend von einem kartenförmigen Grundriß ist die Blisterpackung 2 zu einem Hohlstab 5 gefaltet. Die Faltung geht quer zur längeren Seite der Blisterpackung 2, und zwar um eine Längsmittelachse x-x. Wie Fig. 12 entnehmbar, sind vier flächengleiche Längsstreifen über Faltlinien 6 definiert. Hierdurch kommt man zu einem mehrseitigen Querschnitt, hier zu einem quadratischen.

Die zu einem Kastenprofil führende Faltrichtung ist so gewählt, daß die Kavitäten 1 in das Innere des Blisterpackungs-Stabes 5 ragen.

Bezüglich der dargestellten Kavitäten 1 handelt es sich um napfförmige Einziehungen 7 einer dünnen Aluminium/Kunststoff-Verbundfolie 8.

Ausrüstungsmäßig ist darauf geachtet, daß auf allen vier gebildeten Breitseiten der Längsstreifen je eine lineare Reihe an Kavitäten 1 vorliegt. Konkret handelt es sich um sieben gleich längsbeabstandete Kavitäten 1. Der Rapport ist auf allen Längsstreifen gleich.

Die Kavitäten 1 sind durch eine durchstoßbare Deckfolie 9 verschlossen. Das enthaltene Pulver, dargestellt als Punkteraster, ist mit 10 bezeichnet. Es nimmt räumlich nur ein Teilvolumen der Kavität 1 ein.

Der Blisterpackungs-Hohlstab 5 wird in Längsrichtung des Trägers T gehend eingeschoben, und zwar gemäß Zeichnung von der linken Seite der Vorrichtung V her. Unter Berücksichtigung des quadratischen Querschnitts des Blisterpackungs-Hohlstabes 5 läßt sich dieser leersauggerecht jeweils um 90° um seine Längsmittelachse x-x wenden, den Bedarf von vier Wochen abdeckend.

Das restfreie Leersaugen der pulverenthaltenden Kavitäten 1 geschieht über ein Saugrohr 11. Das ist so gestaltet, daß es mit seinem vorderen, der Kavität 1 zugewandten Ende die Deckfolie 9 durchstößt. Hierzu weist das Saugrohr 11 eine Schneide 12 in Form einer zentralliegenden blattartigen Flanke auf. Letztere ist in Form eines Bügelmessers gestaltet und durchstößt den freigespannten Abschnitt 9' der Deckfolie 9 der Kavität 1. Die diesbezügliche Ausgestaltung ist im einzelnen so, daß die zentralliegende Flanke von einem über eine abgeplattete Stirnfläche 13 des Saugrohres 11 vorstehenden Bügel mit spitzdachförmig zueinander stehenden Schneidenflanken gestaltet ist. Die Spitze liegt mittig.

Dieser mittelständigen, schneidenbildenden Flanke sind radial (seitlich) benachbarte Flanken als Stößelblätter 14 zugeordnet. Diese verdrängen die freigeschnittenen Flächenabschnitte der Deckfolie. Sie sind etwas kürzer als die Schneide 12 und weiten den Schnittspalt des freigespannten Abschnitts 9'. Weitere Details sind der nicht veröffentlichen deutschen Patentanmeldung 197 57 207.3 entnehmbar. Deren Offenbarungsinhalt wird hier vollinhaltlich mit einbezogen, auch zu dem Zweck, Merkmale dieser Unterlagen in die Ansprüche vorliegender Anmeldung mit aufzunehmen.

Eine günstige Zuordnung des Saugrohres 11 am Träger T wird über die dargestellte Längsführung 15 erreicht. Die ist auf ein schlittenförmiges, lineares Verfahren des Saugrohres 11 eingerichtet. Der stabförmige Träger T weist dazu zugangsmäßig an einer Seitenfläche 16 einen Durchbruch auf. Der ist als geradlinige Reihe von Saugrohr-Durchtritts-Löchern 17 realisiert. Besagte Löcher 17 liegen kongruent zu den sich darunter erstrekkenden Blisterpackungs-Kavitäten 1. Die Löcher 17 stehen über wespentaillenartige Einziehungen miteinander in Verbindung, dies in Art eines gewellte Flanken aufweisenden Langloches. Die entsprechend schmäleren Querschnitte zwischen jeweils zwei Löchern 17 tragen das Bezugszeichen 18.

Die spurgebende Längsführung 15 für das Saugrohr 11 besteht aus beiderseits der Reihe von Löchern 17 liegenden Schlitzen 19 des Trägers T. Die Schlitze 19 verlaufen parallel und möglichst randnah der Löcher 17 bzw. bei nur einer Lochreihe randnah der Seitenfläche 16. So ergibt sich eine gute, verkantungsfreie Führung für das Saugrohr 11. Die parallel ausgerichteten Schlitze 19 sind im Bereich der Ladeöffnung 20 geschlossen und im Bereich des anderen Stirnendes für die Montage offen. Hier gilt es aber einen leicht schlitzverschmälernden Rastnocken 21 zu überwinden, so daß dieser Bereich normalerweise als Anschlag empfunden wird, der bei moderaten Kräften auch so wirkt.

Den geführten Part des Saugrohres 1 stellen in den Schlitzen 19 laufende Fortsätze in Form von Laschen 22 dar. Die sind dem Saugrohr 11 beiderseits des von ihm gebildeten Schneidkopfes leitblechartig angeformt. Die Führungslaschen 22 erstrecken sich parallel und tauchen relativ tief in die Schlitze 19 ein. Sie sind den Schneiden und Stößelblättern 14 mit Abstand benachbart und stehen axial nach vorne über die Stößelblätter und Schneide vor.

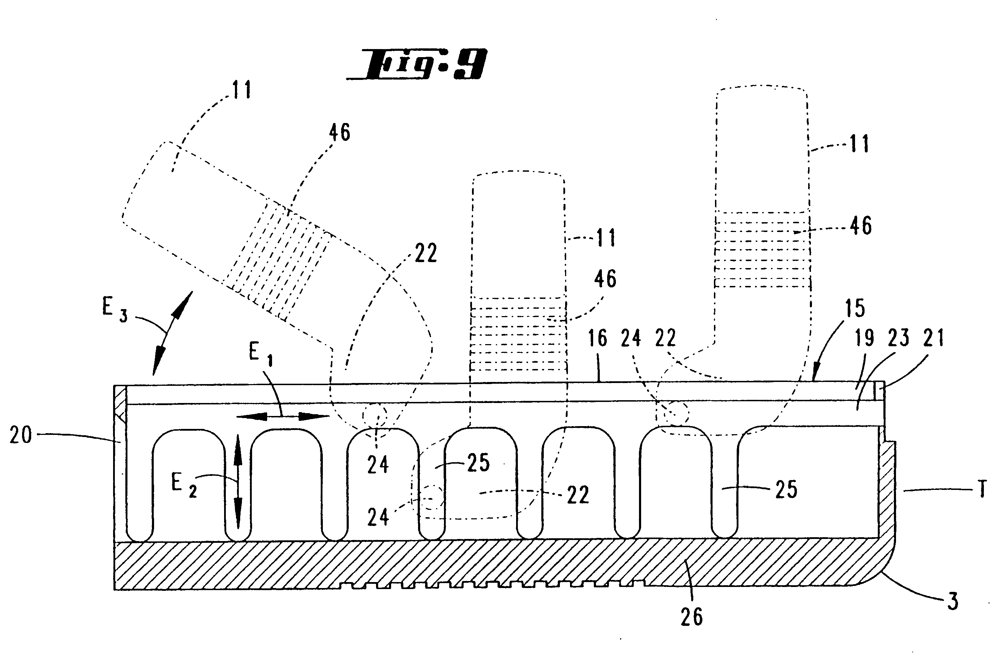

Unterhalb der Schlitze 19 erstrecken sich in der Wandung der beiden anschließenden Seitenflächen des Trägers T Längsnuten 23. Es sei auch auf Figur 9 verwiesen.

In den Längsnuten 23 gleiten nach auswärts gerichtete, den freien Enden der Laschen 22 angeformte Nocken 24. Die führen und fesseln zugleich das Saugrohr 11 unverlierbar am Träger T.

Die besagten Nocken 24 sind räumlich so angeordnet, daß sich das Saugrohr 11 in eine abgestützte Stellung genau deckend über die Löcher 17 abklappen läßt. Der in Richtung des Blisterpackungs-Hohlstabs 5 gehende Verlagerungshub des Saugrohres 11 ist zugleich der Perforationshub. Um dieses in Richtung der Kavität 1 gehende Durchstoß-Verlagern zu erreichen, sind unterhalb der Schlitze 19 Positionierungsfreiräume 25 ausgeformt. Diese gehen als Stichkanäle von der Nut 23 in Richtung eines von der unteren Wandung des Trägers T gebildeten Bodens 26 aus. In Figur 9 ist eine die Durchstoßstellung wiedergebende Ausrichtung des Saugrohres 11 dargestellt. Es handelt sich um die mittlere Stellung. Die entsprechende Kulissenführung für die parallelen Nocken 24 ist so, daß es auf der Zwischenstrecke zwischen zwei Durchstoßstellungen keine Berührung der Schneide 11 mit Partien des Trägers T oder des Blisterpackungs-Hohlstabes 5 kommt, als nur zu einer perforationsgerechten, formschlüssig undrehbarer Vertikalführung für den sich stechseitig in das Bügelmesser fortsetzenden Stößelabschnitt 27 des Saugrohres 11.

Erreicht ist das Ganze durch eine Führung zwischen Saugrohr 11 und Träger T derart, daß das Saugrohr 11 nacheinander in zwei zueinander liegenden Ebenen beweglich ist. Bezüglich dieser Ebenen handelt es sich um eine horizontale Ebene E1, verantwortlich für den Positionssprung zur nächsten Kavität und um eine anschließende vertikale Ebene E2, in der in der Endphase das Öffnen der Kavität 1 vorgenommen wird. Diese beiden Ebenen E1 und E2 sind durch Doppelpfeile angegeben. Die Ebenen kommen durch entsprechenden Spurabgriff zwischen Nocken 24 und Nut 23 und Stichkanäle zustande.

Schneide und Stößelblätter liegen zurückversetzt und geschützt auch bei Nichtgebrauch, z.B. einer weiter unten erklärten Abklappstellung des Saugrohres 11. Dabei ist eine vorteilhafte Ausgestaltung auch noch dadurch erreicht, daß in einer der beiden Ebenen E1, E2 das Saugrohr sowohl klappbar als linear geführt verschieblich ist. Das Saugrohr 11 kann auf dem Bauch liegend in der Ebene E1 verschoben werden. Dabei führt sich der Nocken 24 in der Ebene E1. Die Klappbogenebene ist in Figur 9 mit E3 bezeichnet.

Der geführt in die Löcher 17 eintretende Stößelabschnitt 27 ist derart gestaltet bzw. zugeordnet, daß er sich zu einer Linearführung des Saugrohres 11 ergänzt, wenn die Nocken 24 in die Stichkanäle (sprich Positionsfreiräume 25) eintreten und darin geführt bodenwärts gleiten. Sie setzen dort im Bereich der Schlitzenden jedoch nicht auf, da der Endbereich als Rangierzone für die Nocken 24 beim Kippen des Saugrohres 11 fungiert.

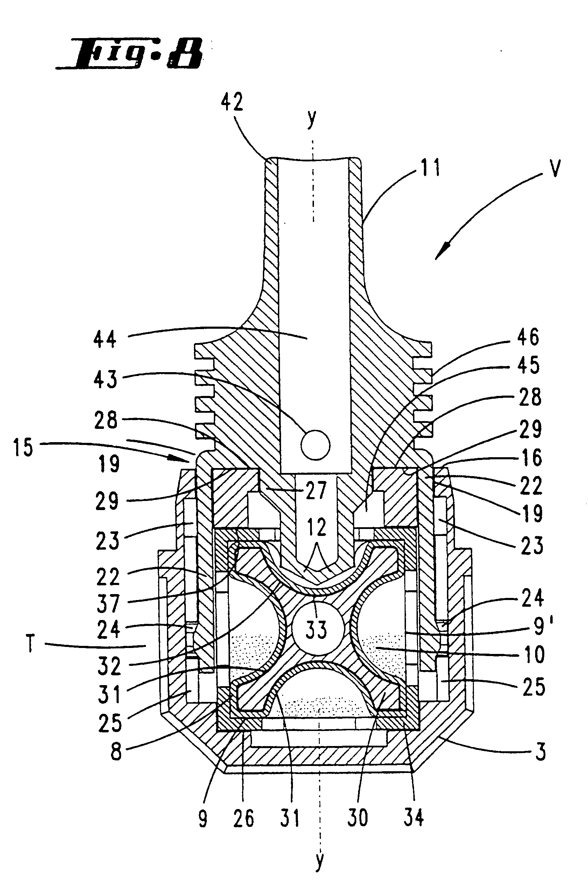

Vorrangig ist vielmehr ein die vertikale Aufstellage sichernde Sitzstellung des Saugrohres 11. Hierzu sind dem Wurzelbereich der Führungslaschen 22 Schultern 28 gegeben. Die erstrecken sich seitlich des stutzenförmigen Stößelabschnitts 27 des Saugrohres 11. Sie verlaufen senkrecht zur in Figur 7 dargestellten, vertikalen Längsmittelachse y-y des Saugrohres 11. Die die Schultern 28 bildenden Flächen setzen in Durchstoßstellung (Figur 8) auf die Bereiche 29 der korrespondierenden Seitenfläche 16, also beiderseits der Löcher 17 des Trägers T planparallel auf. Das zeigt Figur 8.

Der Träger T enthält einen Kernstab 30. Der wurzelt innen in der rechtsseitigen Stirnwand der Vorrichtung V. Er (30) erstreckt sich freiragend nahezu über die gesamte Länge des Trägers T. Er taucht in den konturentsprechenden Innenquerschnitt 31 (vgl. Fig. 14) des eingeschobenen Blisterpackungs-Hohlstabes 5 ein.

Mindestens eine dem Saugrohr 11 zugewandte Flanke 32 fungiert als Stützboden für die dortige Kavitäten-Unterseite 33. Bevorzugt sind alle vier winkelgleich versetzten Flanken des Kernstabes 30 unter Bildung eines solchen Stützbodens geformt. Es handelt sich um der domförmigen Kontur der Einziehungen 7 silhouetten-entsprechend gestaltete Längsrinnen. Zugrunde liegt ein wendesymmetrisches Profil.

Bei nicht geschlossenem Faltprofil des Blisterpakkungs-Stabes 5 kann dieser ummantelt sein. Das verkörpert sich gemäß zeichnerischer Darstellung in der Zuordnung eines Käfigs 34. Der ist als quadratischen Querschnitt aufweisende Stecktasche realisiert. Obwohl eine reibungsschlüssige Zuordnung beider Teile 5, 34 denkbar ist, sieht die erfindungsgemäße Lösung eine Verrastung vor. Die entsprechende Verrastung zwischen Blisterpakkungs-Hohlstab 5 und Käfig 34 geschieht in der Einschubendstellung des Blisterpackungs-Hohlstabes 5.

Es kann sich bezüglich der Rastmittel patrizenbezogen um eine Warze 35 handeln, die in eine kongruent liegende Vertiefung oder Durchbrechung 36 des Blisterpackungs-Hohlstabes 5 einschnäppert. Je nach Einsatzziel des Käfigs 34, also ob er als Einweg- oder dauerverwendbares-Teil vorgesehen ist, kann es sich um eine reversible oder irreversible Verrastung 35/36 handeln. Für eine Dauerverwendung des Käfigs 34 spricht die technische Grundkonzeption, die Blisterpackung 2 durch den Verbraucher in die Hohlstabform überführen zu lassen. Hier könnte die kartenförmige Blisterpackung 2 raumsparend gestapelt zugeliefert werden.

Alle vier Seitenwände 37 des Einschubkäfigs 34 sind mit Öffnungen 38 und sie verbindenden freien Querschnitten 39 versehen. Es liegt eine Langlochkontur vor, wie sie in bezug auf den Träger T eingehend beschrieben ist. Die Maske ist identisch. So kann die Schneide des Stößelabschnitts 27 auch dieses deckungsgleich liegende Langloch durchgreifen und die darunterliegenden Kavitäten 1 ungehindert erreichen, da die Öffnungen 38 plus Querschnitten 39 mit den Löchern 17 plus Querschnitten 18 flächenmäßig übereinstimmen. Die Stößelblätter 14 sind etwas schmaler als die Querschnitte 39.

Mit oder ohne Einschubkäfig 34 endet die durchstoßende Schneidflanke sprich Schneide 12 schneidenschonend kurz oberhalb der bodenbildenden Innenwand der Kavität 1 bzw. der darunterliegenden Stützbodenfläche d.h. Flanke 32 des Kernstabes 30 des Einschubkäfigs 34.

Der Einschubkäfig 34 ist linksseitig durch eine Stirnwand 40 geschlossen. Letztere bildet innenseitig den Einsteckbegrenzungsanschlag für den Hohlstab 5, wenn keine Rastvorrichtung 35/36 vorliegt.

Besagte Stirnwand 40 überragt sodann allseitig den Querschnitt des stabförmigen Mantelabschnitts des Käfigs 34, so daß hieraus außenseitig ein Einsteckanschlag im Bereich der Ladeöffnung 20 des Trägers T nutzbar ist.

Die Stirnwand 40 übt jedoch auch noch eine weitergehende Funktion aus: Die besteht darin, daß der besagte periphere Überstand durch den Deckel 4 der Vorrichtung V deckelseitig sperrend übergreifbar ist. Die übergreifende Stirnwand des Deckels 4 heißt 41. Sie sichert die Einheit 5/34 so bei Nichtgebrauch gegen ein Herausrutschen aus dem Träger T.

Bei geschlossener Vorrichtung V erstreckt sich das abgeklappte Saugrohr 11 deckelüberfangen aufliegend auf der obenliegenden Seitenfläche 16 des Trägers T. In dieser Stellung befinden sich die Nocken 24 in den Längsnuten 23.

Der Deckel 4 weist eine deutlich konvex gewölbte Decke auf mit in der Querschnittsmitte liegendem Zenit. Dieser Bereich ist für die raumsparende Abklappstellung des Saugrohres 11 so der günstigste.

Wie Figur 6 entnehmbar, nimmt die mundgerecht flache Wandung der Mundstücköffnung 42 im Querschnitt einen elliptischen Wandungsverlauf ein. Die längere Ellipsenachse verläuft in Längsrichtung des Trägers T. Hierdurch hat man eine mundgerechte Position dergestalt, daß man die Vorrichtung V wie eine Mundharmonika in der Hand halten kann, um so bequem den Saughub durchführen zu können.

Das Leersaugen der pulverenthaltenden Kavität 1 geschieht in Unterstützung eines das Pulver 10 mitschleppenden Neben-Luftsaugstromes Z, der über Querbelüftungslöcher 43 des Saugrohres 11 zugeht. Diese erstrecken sich diametral gegenüberliegend und nehmen innen Strömungsanschluß an den Hauptkanal 44 des Saugrohres 11. Durch enge Nähe zur Seitenfläche 16 und eine außenseitige reiche Randzonenklüftung sind die Querlüftungslöcher 43 weder durch Greiffinger der Bedienungshand noch durch Partien der Mundlippe zuhaltbar. Nebenluft tritt im übrigen über das ganze und recht groß zu haltende Umfeld 45 des Stößelabschnitts 27 hinzu. Zur Erhöhung der Griffigkeit des Saugrohres 11 ist dieses mantelflächenseitig gerauht, d.h. ringgerippt. Die Rippung ist mit 46 bezeichnet.

Die Sichtseite der im Grunde quadratischen Stirnwand 40 des Käfigs 34 ist mit Benutzungssymbolen 47 versehen. Es handelt sich um Nummern eins bis vier. Hieran kann der Benutzer ablesen, welche Kavitäten-Reihe noch als Vorrat vorhanden ist, bei angewendeter numerisch ansteigender Umsteckfolge natürlich.

Weiter ist erkennbar, daß die besagte Stirnwand 40 zentralliegend ein quadratisches Plateau 48 besitzt. Dieses exponierte Plateau 48 wirkt bezüglich seiner parallel zum Quadrat liegenden Seitenwände mit dem in Richtung des Gehäuses 3 gerichteten Rand der Stirnwand 41 des Deckels 4 zusammen. Hierüber liegt eine Art Schlüsselfunktion vor. Der "Schlüssel" kann variiert werden, so daß jeweils nur die für die Behandlung bestimmten Medikamente bzw. Magazine zum Einsatz kommen.

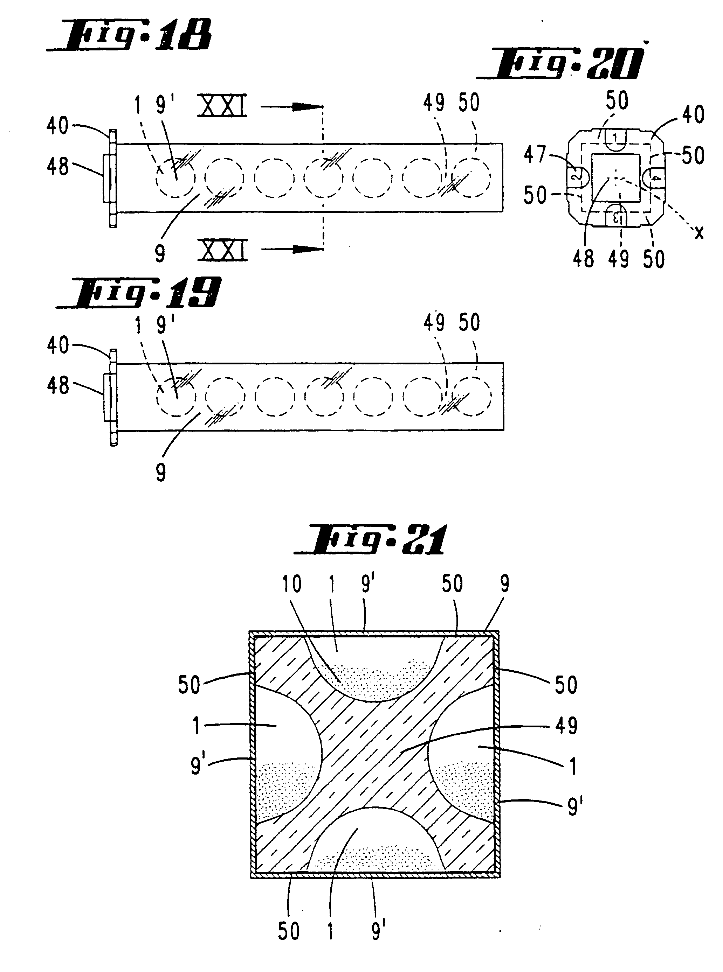

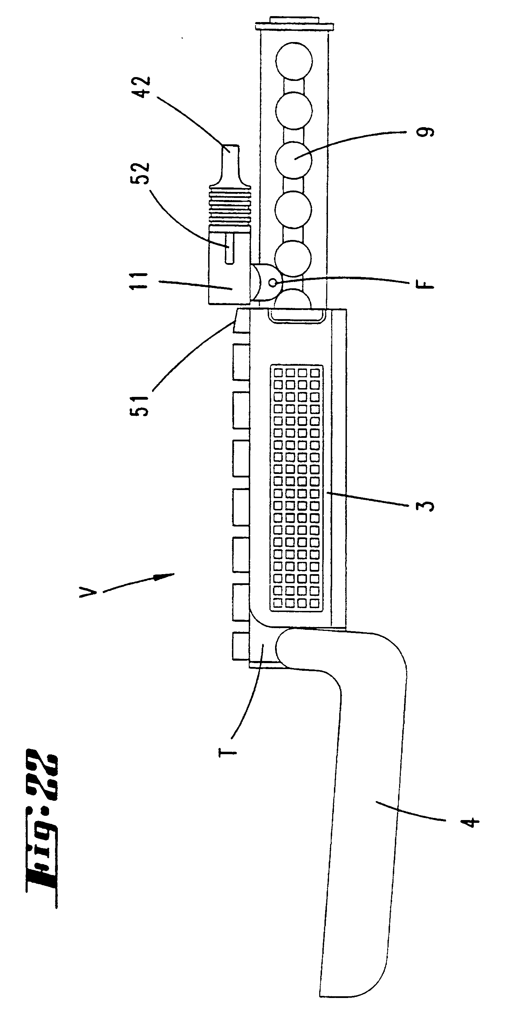

Die geschilderten Vorgaben treffen auch auf das in den Figuren 18 bis 21 wiedergegebene Massivmagazin zu. Dieses Magazin ist aus sich selbst versteift und nicht unter Zuordnung des oben beschriebenen Käfigs 34. Erreicht ist die entsprechende Versteifung durch einen die Blisterpackungs-Kavitäten 1 formenden Stab als Kernstück 49. Letzteres weist quadratischen Querschnitt auf. Dieses das Massivmagazin bildende Kernstück 49 läßt sich in der geschilderten Weise wenden. Es ist auch querschnittsmäßig abgestellt auf einen betriebsgerechten Einschub in den Träger T der Vorrichtung V.

Die Seitenflächen 50 des stabförmigen Kernstücks 49 sind mit der üblichen Deckfolie 9 belegt. Diese kann durch Klebung aufgebracht werden. Die Bezugsziffern sind sinngemäß angewandt.

Wie Figur 21 verdeutlicht, sind die Blisterpackungs-Kavitäten 1 jeweils auf einer gemeinsamen Querebene des Kernstückes 49 angeordnet. Das gilt für die gesamte Kavitätenreihe.

Das Kernstück 49 besteht aus Glas. Ebenso ist seine Fertigung aus Keramik denkbar oder hoch kristallinem Kunststoff.

Bei der abgewandelten Lösung der Figur 22 sitzt das Saugrohr 11 auf dem Blisterpackungs-Stab 5 der auch massiv wie das Kernstück 49 ausgebildet sein kann. Dazu hat die eine Flachseite des Saugrohres 11 z.B. zwei Zapfen, die reibschlüssig in Löcher des Stabes eintreten. Schiebt man den Stab 5 in den Träger, fangen sich die Führungsvorsprünge F in Nuten an den Innen-Seitenwänden des Trägers T ein und gleichzeitig wird das Saugrohr über die Schrägen 51 durch Auflaufen z.B. der Rippen 52 angehoben unter Trennung der Zapfen/Lochverbindung. Jetzt ist das Saugrohr am Träger T geführt und kann geschwenkt werden, um in die Bereitschaftsstellung zum Durchstecken des Folienabschnitts zu kommen. Die seitlichen Rippen 52 des Saugrohres 11 führen sich beim Niederfahren (Durchstechen) in den Zahnlücken 53 des Trägers T.

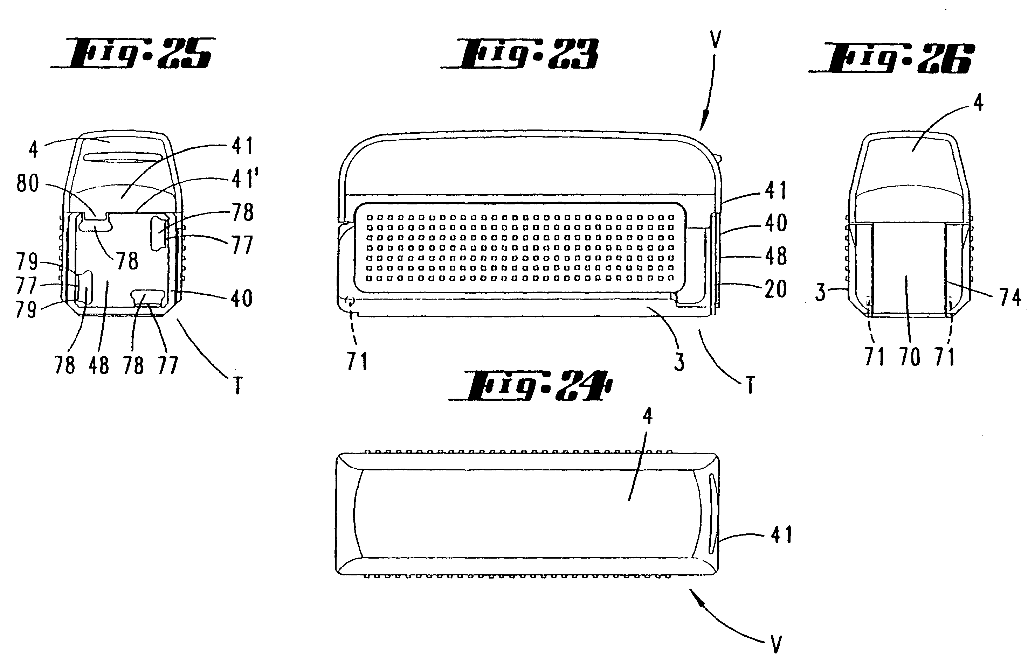

Die auf der abgewandelten Lösung gemäß zweitem Ausführungsbeispiel fußende Weiterbildung der Vorrichtung V ab Fig. 23 wird nachstehend detaillierter erläutert. Dabei sind die Bezugsziffern, soweit Übereinstimmung mit den vorbeschriebenen Konstruktionen besteht, sinngemäß angewandt, dies zum Teil ohne textliche Wiederholungen.

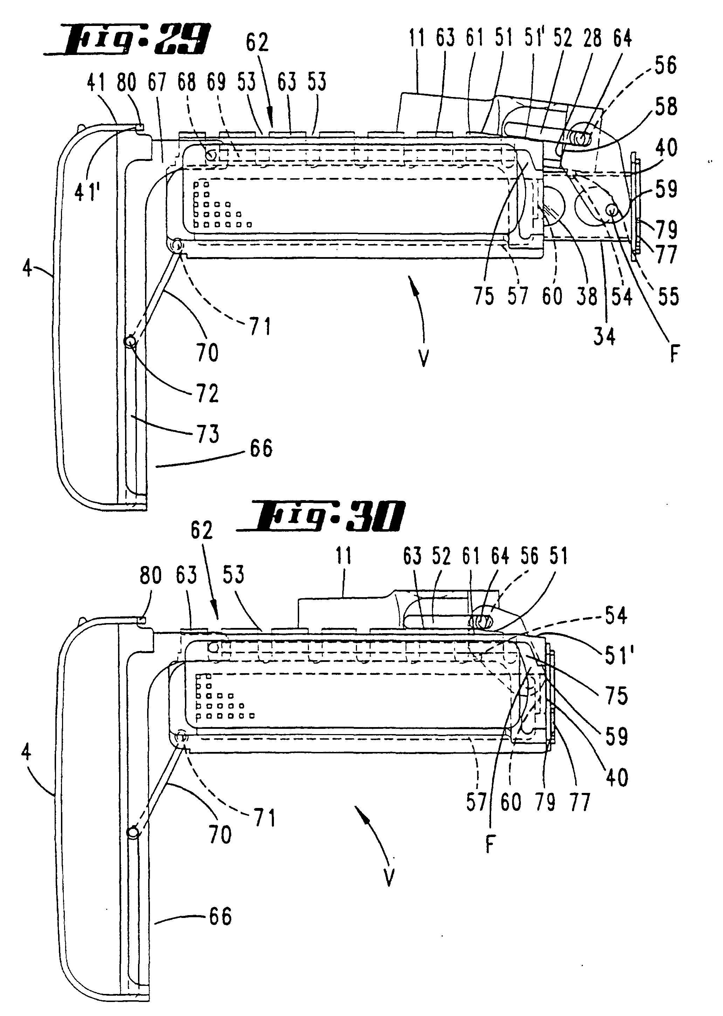

Aus den Fig. 27 bis 30 ist nun auch ein Andockmittel zwischen Saugrohr 11 und dem Stab 5, genauer dem Käfig 34 zu erkennen. Die an der einen Flachseite des Saugrohres 11 senkrecht abstehenden Zapfen tragen das Bezugszeichen 54. Sie stecken bei flach auf die Oberseite aufgelegtem Saugrohr in kongruent angeordneten Höhlungen oder Löchern 55 des Käfigs 34. Es liegt ein willensbetont überwindbarer Reibschluß vor.

Der stabbestückte Käfig 34 bildet auch hier das Vehikel für eine Übergabe des Saugrohres 11 an den Träger T.

Von den beiden angrenzenden, raumparallel liegenden Flachseiten des Saugrohres 11 gehen auch hier Führungslaschen 22 aus, die gemäß Weiterbildung jedoch gelenkig dem Saugrohr 11 zugeordnet sind. Die entsprechende Querachse trägt das Bezugszeichen 56. Sie liegt ebenenmäßig in der Quermittelebene des symmetrisch gestalteten Saugrohres.

Die Laschen 22 sind wendesymmetrisch gestaltet und erlauben das Umklappen des Saugrohres 11 um die Querachse 56 gehend, so daß das Saugrohr sowohl eine Abklapprichtung einnehmen kann, in der seine Mundstücköffnung 42 in Steckzuordnungsrichtung des Käfigs 34 weist, als auch in Gegenrichtung.

Die Übergabe geschieht im Bereich einer gehäuseseitigen Führung, realisiert als Nuten 57 an den Innenseitenwänden des Trägers T. In diese Nuten 57 greifen Führungsvorsprünge F der Laschen 22 ein. Es handelt sich um abragende zylindrische Stummel. Die befinden sich an den freien Enden der beiden Laschen 22. Ihre Lage in bezug auf die Ladeöffnung 20 des Gehäuses 3 ist definiert. In der Abklappstellung des Saugrohres 11 tritt nämlich dessen Schulter 28 gegen eine Gegenschulter 58 der Laschen 22, die ihrerseits so ein Widerlager an der kragenbildenden Rückseite der Stirnwand 40 des Käfigs 34 finden. Das laschenseitige Ende ist mit 59 bezeichnet. Beiderseits einer gedachten, den Führungsvorsprung F und die Querachse 56 kreuzenden Linie ist aufgrund des wendesymmetrischen Aufbaues in gleicher Weise eine zweite Gegenschulter 58 und ein zweites Anschlag-Ende 59 realisiert. Die Linie verläuft in einem Winkel von 45° zur Steckrichtung des Käfigs 34.

Aufgrund der in Fig. 27 dargestellten, abgestützten Konstellation behält der Führungsvorsprung F, genauer ihr zylindrischer Stummel, eine Längsführungsausrichtung bei, in deren Weg ein in beiden Richtungen willensbetont überwindbarer Rastvorsprung 60 liegt. Besagter Rastvorsprung ist der Nut 57 vorgelagert. Mit Passieren des Rastvorsprunges 60 ist die Zapfenverbindung 54/55 aufgehoben.

Zieht man hiernach den Käfig 34 samt Stab 5 ab, bleibt das Saugrohr 11 via Laschen 22 am Träger T gefesselt. Es kann nun das Wenden und Wiedereinstecken des Käfigs 34 vorgenommen werden. Ein vollständiges Lösen des Saugrohres ist dagegen auch grundsätzlich möglich, bspw. um den nächsten, ein frisches Saugrohr 11 tragenden Stab 5 bzw. Käfig 34 einzusetzen.

Das Lösen des Saugrohres 11 bei der Übergabe desselben an die Führung sprich Nuten 57 des Trägers T wird über einen einlaufseitigen Hubnocken 61 einer Zahnreihe 62 bewirkt. Deren Zähne 63 lassen zwischen sich Zahnlücken 53. Auch bei der vorliegenden Weiterbildung fungieren die Zahnlücken 53 als senkrecht zur Einschubbewegung des Käfigs 34 ausgerichtete Leitwege für die Durchstechbewegung des Saugrohres 11. Die saugrohrseitigen Führungselemente sind auch hier die Rippen 52. Die Zahnlükken 53 sind entsprechend der Teilung der Blisterpackungs-Kavitäten 1 angeordnet, liegen also in dem diesbezüglichen Rapport.

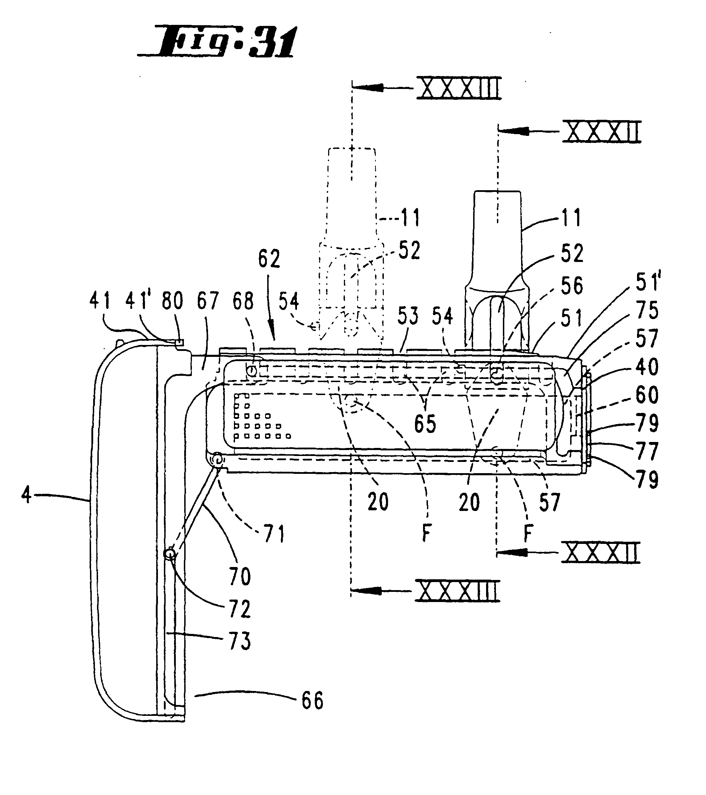

Die sich an dem paarig ausgebildeten Hubnocken 61, letzterer realisiert als kopfseitig abgeschrägter erster Zahn 63 der Zahnreihe 62, anhebenden Rippen 52 kommen im Anschluß an diese in Fig. 29 dargestellte Aushebphase in eine Lage, in der sich die untere Längsseite der geradlinigen Rippen 52 auf den linear ausgerichteten Köpfen der nachfolgenden Zähne 63 der Zahnreihe 62 führt und abstützt. Die Schräge ist auch hier mit 51 bezeichnet. Der Gesamtanstiegsweg am Nocken 61, einschließlich der eines vorgelagerten gehäuseseitigen Nockenabschnitts 51', entspricht der vertikalen Länge des bzw. der Zapfen 54. Es kommt demgemäß zu einem vollständigen Ausheben der Zapfen 54 aus ihren Löchern 55.

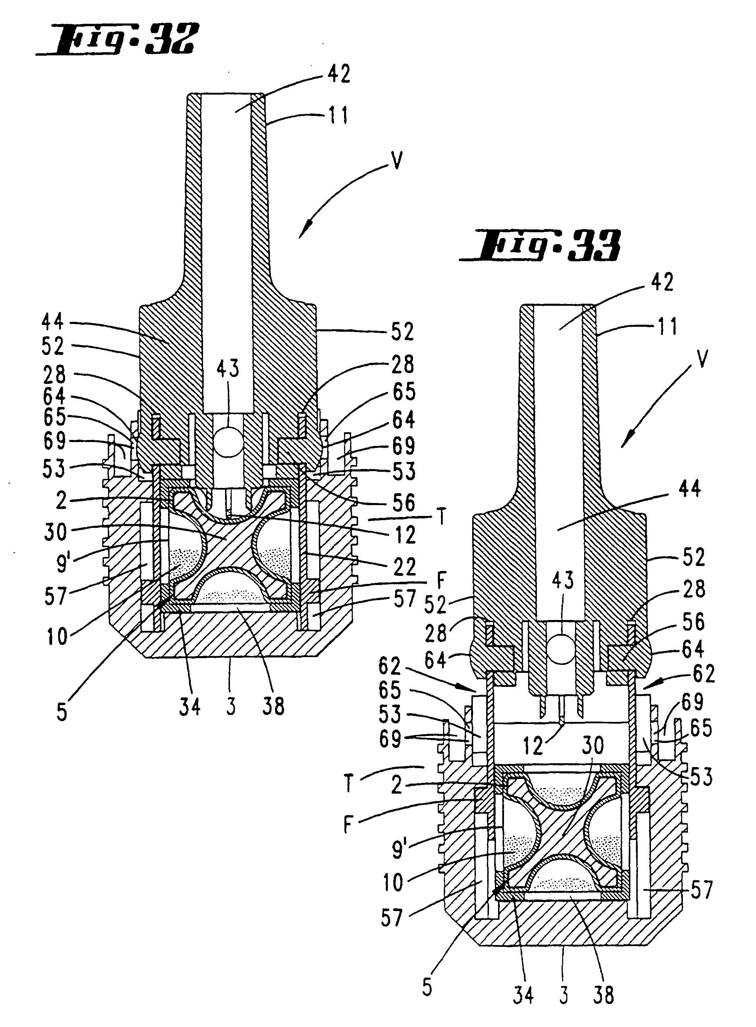

Gemäß Weiterbildung setzt sich die Rippe 52 in Richtung der Querachse 56 außenseitig in einen Rastnocken 64 fort. Der ragt querseitig ab und wirkt mit einer Rastvertiefung 65 zusammen. Die befindet sich im Bereich des Grundes der Zahnlücken 53 (vgl. Fig. 32).

Während in Fig. 22 der kappenförmige Deckel 4 klappbar dem Gehäuse 3 zugeordnet ist - er läßt sich in eine buchdeckelartige Aufklappstellung überführen -, sieht die Weiterbildung gemäß Fig. 23 ff. zusätzlich noch eine Abklappstellung vor. Diese schafft einen das Laden und Entladen und auch die Benutzung begünstigenden oberseitigen Freiraum und sogar einen frontalen Haltegriff 66.

Dazu wird der Deckel 4 im Wege der Klapp-/Schiebebewegung in eine jenseits des rückwärtigen Gehäuseendes, der Ladeöffnung 20 also, liegende Absenkstellung bewegt. Gut zwei Drittel der Länge des Deckels 4 ragt als Haltegriff 66 an der Unterseite des Gehäuses 3 vor.

Das abklappende Öffnen geschieht zwangsgesteuert über ein Gelenkevieleck zwischen Gehäuse 3 und Deckel 4. Der Verlagerungsweg des Deckels 4 ist dabei so, daß der Deckel das in die Abklapplage gebrachte Saugrohr 11 als Überstand über die Oberseite des Gehäuses 3 umrißmäßig noch so berücksichtigt, daß es nicht zu einer Berührung kommt. Im einzelnen ist dabei so vorgegangen, daß der Deckel 4 einen quer abstehenden, paarig gestalteten Ausleger 67 aufweist. Es handelt sich um an der Randseite des kappenförmigen Deckels 4 ansetzende, in der Flucht der Deckel-Längsseitenwände liegende, quer zur Längserstreckung des Deckels abragende, gabelförmige Vorsprünge. Im Endbereich der Ausleger 67 befindet sich ein Führungszapfen 68. Der greift in einen Führungsschlitz 69 des Gehäuses 3 ein. Der geht nahezu über die gesamte Länge des Gehäuses 3 und dient außer der verschieblichen Führung auch der Gelenkbildung, dies im Interesse der angedeuteten Klappbewegung.

Der Führungsschlitz 69 erstreckt sich längs der oberen Gehäuseseite, und zwar die Zahnlücken 53 ebenenmäßig kreuzend, bringend die Rastvertiefungen 65 in Form von fensterartigen Durchdringungen für die Rastnocken 64.

Zusätzlich ist der Deckel 4 über einen Lenker 70 mit dem Gehäuse 3 verbunden, gehäuseseitig über einen festen Anlenkpunkt 71 und deckelseitig über einen verschieblichen Anlenkpunkt 72. Beide sind als Achsstummel realisiert. Zur Verschieblichkeit des Anlenkpunktes 72 weist der Deckel 4 randnah einen parallel zum Deckelrand verlaufenden Längsschlitz 73 auf. Der ist von solcher Länge, daß der Lenker 70 bei in Schließrichtung gegen die dortige Stirnfläche des Gehäuses 3 geklappter Stellung noch ein Weiterlaufen des Deckels 4 in die Schließstellung erlaubt, in der sich die der Ladeöffnung 20 zugehörige Stirnwand 41 des Deckels 4 verschlie-ßend vor die Ladeöffnung 20 legt und dort bspw. verschnäppert. Der Lenker 70 ist dabei in eine stirnflächenseitige Nische 74 des Gehäuses 3 eingeschwenkt.

Der mit 71 bezeichnete Anlenkpunk liegt nahe dem Boden 26 des Gehäuses 3.

Der Führungsschlitz 69 setzt sich am rückwärtigen Gehäuseende in einen abwärtsgerichteten Abschnitt 75 fort. Der reicht nahezu bis zum Boden 26. Er ist quer zur Längserstreckung des Gehäuses 3 als konvex verlaufender Bogen ausgeführt. Der wirkt in bezug auf die Kappenbewegung nach auswärts lenkend, und zwar dergestalt, daß der Abschnitt 75 beim Verlagern des Deckels 4 jeweils eine Hub- bzw. Senkbewegung desselben erzwingt, und zwar so, daß dessen Deckelrandkante 41' das endnah des Käfigs 34 angedockte Saugrohr 11 in abgeklappter Stellung berührungslos überfährt. Es kommt demgemäß also keineswegs zur Berührung des Deckels 4 an der exponierten Kante 76 im Basisbereich des abgeklappten Saugrohres 11.

Die Stirnwand 40 des Käfigs 34 ist im Verein mit der Klappbewegung des Deckels 4 zur Ausbildung einer Benutzungsanzeige bzw. auch Originalitätssicherung ausgebildet. Dazu sind an der Stirnwand 40 mit dem Winkel der Umwendbarkeit des Käfigs 34 übereinstimmend positionierte Originalitätsbrücken 77 realisiert. Sie erstrecken sich im Plateau 48. Sie liegen über den Plateaurand zugänglich. Es handelt sich um den Eingang einwärtsgerichteter Ausnehmungen 78 versperrende Leisten, die über endseitige Sollbruchstellen 79 am Rand der Ausnehmung 78 hängen.

Die Außenseite der Originalitätsbrücken 77 liegt im Aktionsbereich einer Nase 80 des Deckels 4. Bezüglich der Nase handelt es sich um einen die Randkante 41' des Deckels 4 überragenden Vorsprung, der im Endstadium der Schließbewegung des Deckels die Originalitätsbrücke 77 "zum Einsturz bringt", d.h. herausbricht.

Eine solche Originalitätsbrücke 77 kann auffällig, das heißt optisch-visuell kontrastierend zur Farbe der Stirnwand 40 ausgeführt sein. So fällt sofort auf, welche Kavitäten-Reihe benutzt respektive entleert wurde.

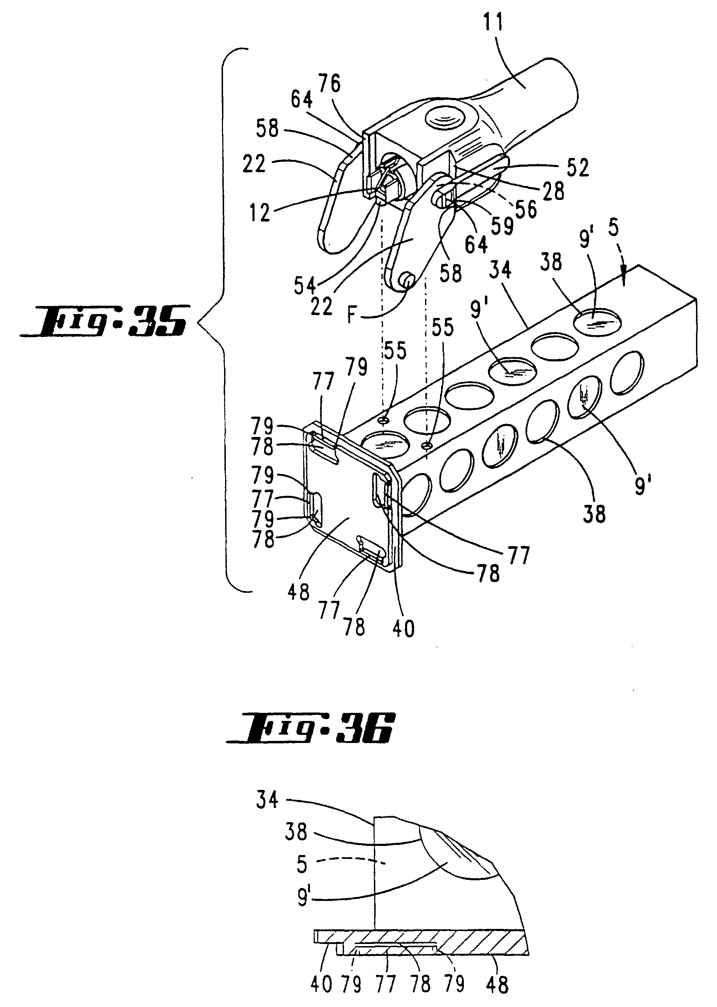

Die Öffnungen 38 im Käfig 34 können isoliert ausgebildet sein, wie das z.B. aus Fig. 34 hervorgeht.

Auch bei der Weiterbildung ist auf eine das Berühren der Schneide 12 vermeidende Verlagerung des Saugrohres 11 geachtet. Die Nuten 57 sind höhenmäßig auf den Vertikalhub des Saugrohres abgestimmt. Vorrangig kommt es stets zu einer führenden Ausrichtung des Saugrohres in den Zahnlücken 53. In dieser Stellung überlagert die Schneide 14 stechgerecht die Deckfolie 9 bzw. den freigespannten Abschnitt 9'.

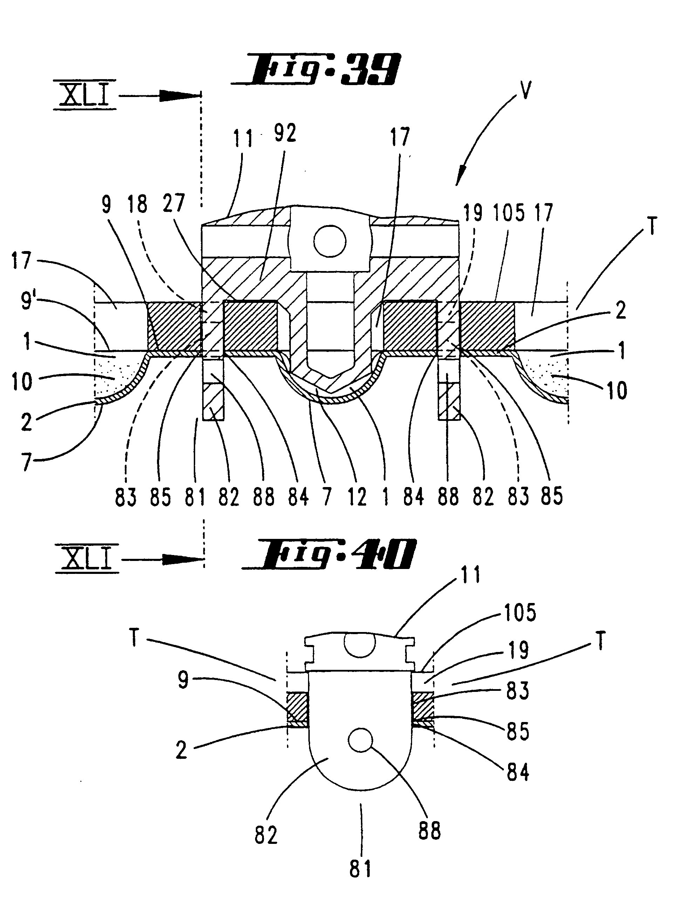

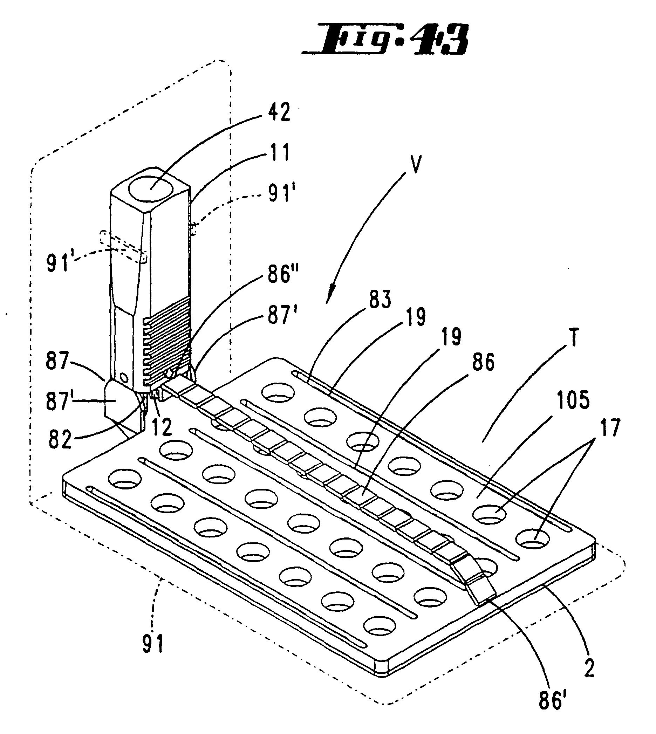

Das dritte Ausführungsbeispiel (Figuren 38 bis 44) zeigt eine baulich abgewandelte Einheit. Lösungsmäßig fußt das auf einer Nutzung der Schlitze 19 des Trägers T der vorerläuterten Ausführungsbeispiele. Dabei wird auch die Laschenpositionierung in die vorliegende technische Konzeption mit einbezogen. Es erstrecken sich Fortsätze 81 mit Abstand seitlich der durchstoßenden Flanken des Saugrohres 11, von denen eine mittlere, auch hier die Schneide 12 bildend, wiederum in Form eines Bügelmessers und die beiden benachbarten Flanken als die Deckfolienlappen zur Seite verdrängende Stößelblätter 14 fungieren.

Die Fortsätze 81 sind in Form von Laschen 82 gestaltet. Die geben dem Saugrohr 11 stechdornseitig die Gestalt eines Gabelkopfes. Die Laschen 82 sind so angeordnet, daß eine Fingerkuppe einer menschlichen Hand nicht in den Gabelraum reicht, es also nicht zu einer diesbezüglichen Kontamination oder Beschädigung an den durchstoßenden Flanken kommt. Die Laschen 82 stehen axial und starr über die Stößelblätter und Schneide über.

Die in einer etwa dem größeren Umriß einer Zigarettenschachtel entsprechenden Platte 105 des Trägers T hat die Löcher 17 in mehreren (z.B. vier) Reihen und noch mehr (z.B. sieben) Zeilen angeordnet und oberseitig parallel zu den Reihen verlaufende Schlitze 19. Diese erstrecken sich als Leitschienen bzw. -nuten seitlich der Reihen der Saugrohr-Durchtrittslöcher 17 und erlauben eine bequeme Vor-Orientierung des Saugrohrs. Die Laschen 82 übernehmen so beim Positionieren und Einstekken des Saugrohres 11 in die Löcher 17 die besagte Funktion.

Die gabelzinkenartigen Fortsätze 81, respektive die sie verkörpernden Laschen 82, sind von einer solchen axialen Vorstandslänge, daß bei in die Schlitze 19 eingesteckter Position die Bügelmesser-Schneide 12 sowie die seitlich benachbarten, etwas kürzeren Stößelblätter 14 die Oberseite der Platte 105 nicht berühren, also nicht beschädigbar sind. Der Eintritt in die Kavität 1 ist erst möglich, wenn die steckgerechte Positionierung des Saugrohres 11 gegeben ist. Diese Position ist definiert durch Einzelschlitze bezeichnet als Schlitze 83 im Träger T und gehen vom Boden der Schlitze 19 aus abwärts. Besagte Schlitze 83 liegen dadurch auch seitlich, gegebenenfalls paarig, der Saugrohr-Durchtrittslöcher 17. Es kommt zu einer paßstiftartigen und damit exakten Andocksituation. Für diesen Zweck alleine könnte auch ein seitlicher Fortsatz 81 ausreichen. Der paarige Aufbau dient jedoch einem weiter unten erläuterten Zweck. Die in der Platte 105 angeordneten Schlitze 83 sind gestalt- und funktionsmäßig auch den oben erläuterten Zahnlücken 53 vergleichbar, in welche dort die seitlichen Rippen 52 des Saugrohres 11 eintreten.

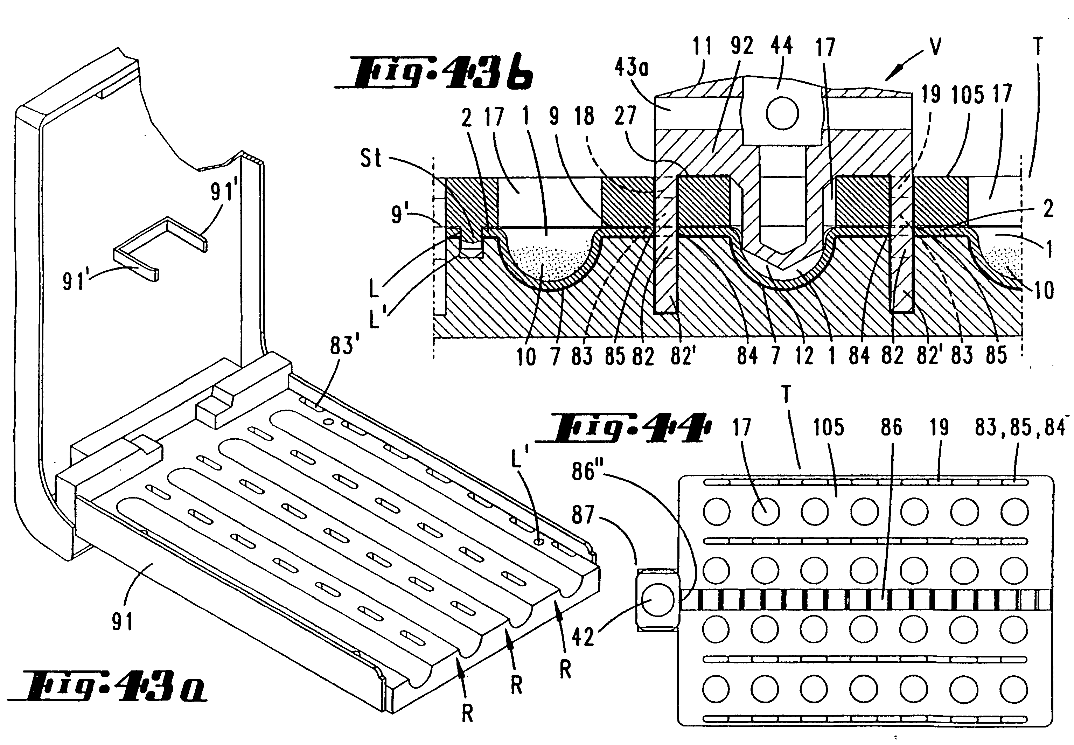

Platte 105 und Blisterpackung 2 sind miteinander verbunden. Die Platte 105 erstreckt sich wie eine Maske oder ein Lochsieb über der Blisterpackung 2. Im Bereich der vier Ecken des Trägers abwärts ragende Stifte St treten fesselnd durch Löcher L des Blisters 2 (siehe Fig. 43b) und eventuell bis in Löcher L' im Unterteil. Die Löcher 17 liegen exakt über den Kavitäten 1. Die vom Grund der Schlitze 19 ausgehenden Einzelschlitze 83 liegen dekkungsgleich zu je einem Einsteckschlitz 84 der Blisterpackung 2. Zweckmäßig ist auch noch die Deckfolie 9 so kongruent geschlitzt. Deren Einsteckschlitz ist mit 85 bezeichnet. Die Schlitze 83,85,84 werden sämtlich von der Lasche 82 durchgriffen. Die tritt endlich freiragend in den Zwischenraum zwischen den die Kavitäten 1 bildenden Einziehungen 7 der Blisterpackung 2. Letztere ist dadurch für die entsprechende Nutzung bezüglich des Saugrohres individualisiert.

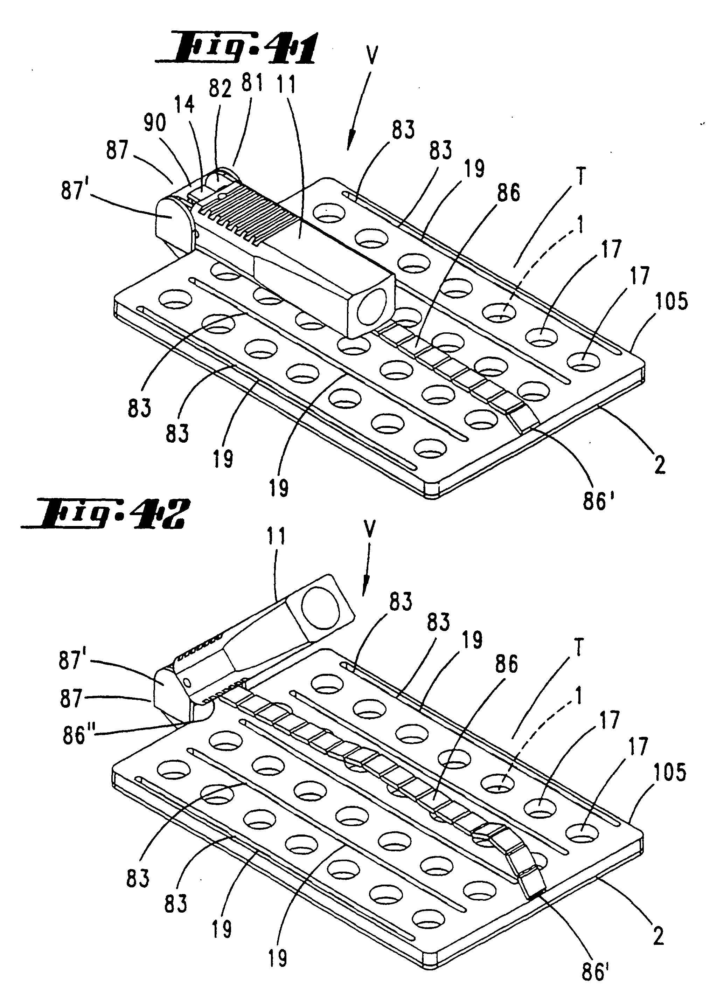

Die Laschen 82 sind endseitig halbkreisförmig konvex verrundet. Das wirkt steckzentrierend auf das Saugrohr 11 und erleichert das Anfahren der Einsteckposition. Im Steckverbund stützen sich die parallelen Längsflanken der Laschen 82 an den Enden der Schlitze bzw. Einsteckschlitze 83 bzw. 85 und 84 ab (vergleiche Figur 40).

Das Saugrohr 11 steht über ein Halteband 86 fest, jedoch versatzbeweglich mit dem Träger T bzw. der Platte oder dem Blister 105 in Verbindung. Bezüglich des Haltebandes 86 handelt es sich um einen panzerkettenartigen Körper, dessen Einzelglieder über Filmscharniere miteinander in Verbindung stehen. Der integrale, stoffschlüssige Verbund liegt bevorzugt mittig an der einen Schmalseite der rechteckigen Grundriß aufweisenden Platte 105. Die Befestigungsstelle des Haltebandes 86 ist mit 86' und die saugrohrseitige mit 86'' bezeichnet. Es handelt sich auch hier jeweils um Filmscharniere.

Das unverlierbar und entsorgungsentsprechend angebundene Saugrohr 11 ist lösbar am Träger T halterbar. Seine Platte 105 weist dazu im Bereich der anderen Schmalseite der Platte 105 eine Halterung 87 auf. Auch die liegt mittig. Zur dortigen Zuordnung sind saugrohrseitig die Fortsätze 81 bzw. Laschen 82 herangezogen. Sie fungieren dort als Gelenklaschen im Sinne von Lageraugen 88. In deren Loch oder Höhlung schnäppern trägerseitige Achsstummel 89 ein. Letztere sind so gelegt, daß sie dem Saugrohr 11 eine klapptechnische Zuordnung am Träger T gewähren. Die warzenartig oder halbkugelförmig ausgebildeten Achsstummel 89 sind den Innenseiten zweier Wangen 87' der Halterung 87 angeformt, welche parallelverlaufenden Wangen 87' zusammen mit einer äußeren, sie verbindenden Endwand 87'' und der gegebenenfalls randausladenden Oberseite der Platte 105 eine Lagertasche 90 bilden. Das Saugrohr 11 läßt sich so in einer die durchstoßenden Flanken schonenden Weise flach auf die Oberseite des Trägers T klappen oder in eine definierte, greifbereite, abragende Stellung überführen. Es sei auf die Figuren 41 bis 43 verwiesen. Die Innenseite der Endwand 87'' stellt den Klapp-Begrenzungsanschlag.

Die entsprechende Klappsteuerung kann über ein die Blisterpackung 2 plus Träger T aufnehmendes, deckelverschlossenes Gehäuse 91 gehen. Es handelt sich um eine Klappschachtel mit Deckel. Die besagte Versorgungseinheit ist dabei so zugeordnet, daß am Saugrohr 11 verrastende Mitnehmer 91' des Deckels das Saugrohr 11, um die durch die Achsstummel 89 definierte geometrische Achse schwenkend, in eine griffbereite Lage hochklappen (Figur 43). In Hochklappstellung gebracht, läßt sich das Saugrohr 11 bezüglich seiner als Schnäpper fungierenden Achsstummel 89 bequem aus der Lagertasche 90 der Halterung 87 ausschnäppern und nach Gebrauch entsprechend wieder zuordnen. Träger plus daran haftendem Blister 2 sind im Unterteil der Klappschachtel angeordnet und dortige bodenseitige Längsrippen R treten jeweils passend zwischen zwei Kavitätenreihen. Im Scheitel haben diese Längsrippen R Einstecköffnungen 83' für die Enden 82' der Laschen 82, welche Einstecköffnungen 83' deckend zu den Einzelschlitzen 83 des Blisters liegen (Fig. 43b).

Die Anbindung des Saugrohres 11 an die Versorgungseinheit stellt einen zeitlich begrenzten Gebrauch desselben sicher, so daß also mit jeder neuen Einheit T/2 bzw. Blister 2 immer wieder ein frisches Saugrohr 11 in Anwendung kommt. Die Bezugsziffern der eingangs erläuterten Ausführungsbeispiele sind, soweit zum Verständnis erforderlich, sinngemäß angewandt, zum Teil ohne textliche Wiederholungen.

## Patentansprüche

1. Vorrichtung (V) zum Entleeren pulverenthaltender und mittels Deckfolie (9) verschlossener Kavitäten (1) mittels eines von Hand setzbaren Saugrohres (11), dessen vorderes Ende relativ zur Kavität (1) positioniert und geführt die Deckfolie (9) durchstößt unter Belassung eines freien Querschnitts zum Einströmen von Luft in die Kavität (1), **dadurch gekennzeichnet, daß** das vordere Ende des Saugrohres (11) benachbart und axial überstehend zu dort vorgesehenen Schneidflanken (12) Fortsätze (22/82) besitzt, die sich in den Kavitäten (1) benachbarten Schlitzen (19 und/oder 83, 55 bzw. 25) eines Trägers (T) formschlüssig/drehfest führen, in welchen Träger (T) die Kavitäten (1) als Blisterpakkungen (2) einsetzbar sind.

2. Vorrichtung nach Anspruch 1 oder insbesondere danach, **gekennzeichnet durch** eine Führung des Saugrohres (11) zum Träger (T) derart, daß das Saugrohr (11) (nur) nacheinander in zwei zueinander liegenden Ebenen (E1, E2) beweglich ist.

3. Vorrichtung nach einem oder mehreren der vorhergehenden Ansprüche oder insbesondere danach, **gekennzeichnet durch** mindestens zwei mit Abstand gegenüberliegende und die Schneid-Flanken in Achsrichtung des Saugrohres (11) überragende Fortsätze (82) des Saugrohres (11).

4. Vorrichtung nach einem oder mehreren der vorhergehenden Ansprüche oder insbesondere danach, **dadurch gekennzeichnet, daß** die Fortsätze (82) als flache Laschen (82) ausgebildet sind.

5. Vorrichtung nach einem oder mehreren der vorhergehenden Ansprüche oder insbesondere danach, **dadurch gekennzeichnet, daß** die Laschen (82) in Schlitzen (19) verschieblich sind und anschließend in Einzelschlitze (83) einfahrbar sind, die vom Grund der Schlitze 19 ausgehen.

6. Vorrichtung nach einem oder mehreren der vorhergehendenden Ansprüche oder insbesondere danach, **dadurch gekennzeichnet, daß** in einer der beiden Ebenen (E1, E2) das Saugrohr (11) sowohl klappbar als auch linear geführt verschieblich ist.

7. Vorrichtung nach einem oder mehreren der vorhergehenden Ansprüche oder insbesondere danach, **gekennzeichnet durch** unterhalb der Schlitze (19) liegende Saugrohr-Positionierungsfreiräume (25/83).

8. Vorrichtung nach einem oder mehreren der vorhergehenden Ansprüche oder insbesondere danach, **dadurch gekennzeichnet, daß** das Saugrohr (11) in beiderseits der Löcher (17) liegenden durchgehenden Schlitzen (19) des Trägers (T) durchgehend verschieblich ist.

9. Vorrichtung nach einem oder mehreren der vorhergehenden Ansprüche oder insbesondere danach, **gekennzeichnet durch** eine zusätzlich schwenkbewegliche Zuordnung der Laschen (82) zu dem Blisterpackungs-Träger (T) bei noch nicht eingestecktem Saugrohr (11).

10. Vorrichtung nach einem oder mehreren der vorhergehenden Ansprüche oder insbesondere danach, **dadurch gekennzeichnet, daß** die Fortsätze (81) Gelenklaschen bilden durch Eintritt trägerseitiger Achsstummel (89).

11. Vorrichtung nach einem oder mehreren der vorhergehenden Ansprüche oder insbesondere danach, **dadurch gekennzeichnet, daß** bogenförmige Stirnkanten der Laschen als Führungen beim Positionieren und Einfahren der Schneide des Saugrohres (11) in die Löcher (17) dienen.

12. Vorrichtung nach einem oder mehreren der vorhergehenden Ansprüche oder insbesondere danach, **dadurch gekennzeichnet, daß** die in Schlitze (19) des Blisterpakkungs-Trägers (T) eintretenden Laschen (22) und das Saugrohr (11) in dieser Stellung deckend über die Löcher (17) klappbar ist.

13. Vorrichtung nach einem oder mehreren der vorhergehenden Ansprüche oder insbesondere danach, **dadurch gekennzeichnet, daß** die Saugrohr-Positionierungsfreiräume(25) als von den Schlitzen (19) ausgehende Stichkanäle ausgebildet sind und die Laschen (22) je einen Nocken (24) aufweisen, die vom Schlitzbereich in die Stichkanäle einfahrbar sind.

14. Vorrichtung nach einem oder mehreren der vorhergehenden Ansprüche oder insbesondere danach, **dadurch gekennzeichnet, daß** das Saugrohr (11) einen in die Löcher (17) einfahrenden Stößelabschnitt (27) besitzt, der zurückversetzt zu der durchstoßenden Schneid-Flanke derart angeordnet ist, daß er sich zu einer Linearführung des Saugrohres (11) ergänzt, wenn die Nocken (24) in die Stichkanäle eintreten.

15. Vorrichtung nach einem oder mehreren der vorhergehenden Ansprüche oder insbesondere danach, **dadurch gekennzeichnet, daß** dem stutzenförmigen Stößelabschnitt (27) benachbarte Schultern (28) des Saugrohres (11) in Durchstoßendstellung auf Bereiche (29) seitlich der Löcher (17) des Trägers (T) aufsetzen.

16. Vorrichtung nach einem oder mehreren der vorhergehenden Ansprüche oder insbesondere danach, **dadurch gekennzeichnet, daß** die durchstoßende Schneidflanke (Schneide 12) bis kurz oberhalb der Bodenfläche der Kavitäten (1) der Blisterpackung (5) reicht und von weniger weit reichenden Stößelblättern (14) benachbart ist.

17. Vorrichtung nach einem oder mehreren der vorhergehenden Ansprüche oder insbesondere danach, **gekennzeichnet durch** eine mittels eines Deckels klappbare Halterung für die Aufbewahrung des Saugrohres (11).

18. Vorrichtung nach einem oder mehreren der vorhergehenden Ansprüche oder insbesondere danach, **gekennzeichnet durch** einen das abgeklappte Saugrohr (11) überfangenden Deckel (4) des Trägers (T).

19. Vorrichtung nach einem oder mehreren der vorhergehenden Ansprüche oder insbesondere danach, **dadurch gekennzeichnet, daß** der Deckel (4) mit seiner schmalseitigen Stirnwand (41) die Einschiebestellung der Blisterpackung (2) sichert.

20. Vorrichtung nach einem oder mehreren der vorhergehenden Ansprüche oder insbesondere danach, **dadurch gekennzeichnet, daß** der die Blisterpackung (2) aufnehmende Träger (T) sich in eine Halterung (87) fortsetzt, in welche die ausrastbaren Achsstummel (89) eintreten, insgesamt mit der Blisterpackung (2) in ein deckelverschlossenes Gehäuse (91) einlegbar ist derart, daß am Saugrohr (11) verrastende Mitnehmer (91') das Saugrohr (11) um die Achsstummel (89) schwenkend in eine griffbereite Lage hochklappen.

21. Vorrichtung nach einem oder mehreren der vorhergehenden Ansprüche oder insbesondere danach, **gekennzeichnet durch** eine kammartig vorstehende Zahnreihe (62) an jeder Seitenwand des Gehäuses (3), in dessen Zahnlücken (53) sich das Saugrohr (11) bei der Durchstechbewegung des Saugrohres (11) führt.

22. Vorrichtung nach einem oder mehreren der vorhergehenden Ansprüche oder insbesondere danach, **dadurch gekennzeichnet, daß** die vertikalgerichtete Durchstechbewegung des Saugrohres (11) geführt ist über Rippen (52) des Saugrohres (11), die in den Zahnlücken (53) des Gehäuses (3) laufen, welche Zahnlücken (53) entsprechend der Teilung der Blisterpackungs-Kavitäten (1) angeordnet sind.

23. Vorrichtung nach einem oder mehreren der vorhergehenden Ansprüche oder insbesondere danach, **dadurch gekennzeichnet, daß** das Saugrohr (11) in der niedergesteckten Stellung verrastet (64, 65).

24. Vorrichtung nach einem oder mehreren der vorhergehenden Ansprüche oder insbesondere danach, **dadurch gekennzeichnet, daß** das Saugrohr (11) zufolge gelenkiger Anordnung an seinen Laschen (22) um seine Querachse (56) umwendbar ist.

25. Vorrichtung nach einem oder mehreren der vorhergehenden Ansprüche oder insbesondere danach, **dadurch gekennzeichnet, daß** die paarig angeordneten Laschen (22) wendesymmetrisch gestaltet sind und sich in Abklappstellung des Saurohres (11) an den Schultern (28) desselben abstützen.

26. Vorrichtung nach einem oder mehreren der vorhergehenden Ansprüche oder insbesondere danach, **dadurch gekennzeichnet, daß** der flächenförmige Träger (T) für die Blisterpackung (2) Einzeleinsteckschlitze (84,85) zum Eintritt der Laschen (82) besitzt.

27. Vorrichtung nach einem oder mehreren der vorhergehenden Ansprüche oder insbesondere danach, **gekennzeichnet durch** eine Blisterpackung (2), die einschließlich Deckfolie (9) deckend zu den Schlitzen (83) im Träger (T) und seitlich benachbart zu den Kavitäten (1) Einzel-Einsteckschlitze (84,85) besitzen zum Eintritt der Fortsätze (82).

28. Vorrichtung nach einem oder mehreren der vorhergehenden Ansprüche oder insbesondere danach, **dadurch gekennzeichnet, daß** die Länge der Einzeleinsteck-Schlitze (84,85) der Breite der laschenförmigen Fortsätze (82) entspricht.

29. Vorrichtung nach einem oder mehreren der vorhergehenden Ansprüche oder insbesondere danach, **dadurch gekennzeichnet, daß** der Träger (T) stabförmig gestaltet ist und an einer Seitenfläche (16) eine geradlinige Reihe von Saugrohr-Durchtritts-Löchern (17) aufweist, welche Löcher (17) deckend liegen zu den Blisterpakkungs-Kavitäten (1).

30. Vorrichtung nach einem oder mehreren der vorhergehenden Ansprüche oder insbesondere danach, **dadurch gekennzeichnet, daß** die Löcher (17) durch schmälere freie Querschnitte (18) miteinander verbunden sind.

31. Vorrichtung nach einem oder mehreren der vorhergehenden Ansprüche oder insbesondere danach, **gekennzeichnet durch** einen die Blisterpackungs-Kavitäten (1) formenden Stab als Kernstück (49), dessen Seitenflächen (50) die Blisterpackungs-Deckfolie (9) aufweisen.

32. Vorrichtung nach einem oder mehreren der vorhergehenden Ansprüche oder insbesondere danach, **dadurch gekennzeichnet, daß** der Blisterpackungs-Stab (5) mehrseiten Querschnitt besitzt mit innenliegenden Kavitäten (1) und in mehreren um seine Längsmittelachse (x-x) umwendbaren Stellungen in den Träger (T) einsetzbar ist.

33. Vorrichtung nach einem oder mehreren der vorhergehenden Ansprüche oder insbesondere danach, **dadurch gekennzeichnet, daß** der Blisterpackungs-Stab (5) in Längsrichtung in den Träger (T) einschiebbar ist.

34. Vorrichtung nach einem oder mehreren der vorhergehenden Ansprüche oder insbesondere danach, **dadurch gekennzeichnet, daß** der Blisterpackungs-Stab (5) quadratischen Querschnitt aufweist und auf allen vier Breitseiten je eine Kavitäten-Reihe hat.

35. Vorrichtung nach einem oder mehreren der vorhergehenden Ansprüche oder insbesondere danach, **gekennzeichnet durch** einen gefalteten Blisterpackungs-Hohlstab (5), der von einem Einschubkäfig (34) umfaßt ist, der an allen seinen Seitenwänden (37) mit der Position der Kavitäten (1) übereinstimmende Öffnungen (38) ausbildet.

36. Vorrichtung nach einem oder mehreren der vorhergehenden Ansprüche oder insbesondere danach, **dadurch gekennzeichnet, daß** der Träger (T) einen in den Innenquerschnitt (31) des Blisterpackungs-Hohlstabes (5) eintauchenden Kernstab (30) aufweist, dessen mindestens dem Saugrohr (11) zugekehrte Flanke (32) als Stützboden für die Kavitäten-Unterseite (33) gestaltet ist.

37. Vorrichtung nach einem oder mehreren der vorhergehenden Ansprüche oder insbesondere danach, **dadurch gekennzeichnet, daß** alle vier Flanken (32) des Kernstabes (30) als Stützboden ausgebildet sind.

38. Vorrichtung nach einem oder mehreren der vorhergehenden Ansprüche oder insbesondere danach, **dadurch gekennzeichnet, daß** der Blisterpackungs-Hohlstab (5) in Einschubendstellung verrastet.

39. Vorrichtung nach einem oder mehreren der vorhergehenden Ansprüche oder insbesondere danach, **dadurch gekennzeichnet, daß** die Kavitäten (1) aller Seitenflächen (50) jeweils auf einer gemeinsamen Querebene des Kernstückes (49) liegen.

40. Vorrichtung nach einem oder mehreren der vorhergehenden Ansprüche oder insbesondere danach, **dadurch gekennzeichnet, daß** das Saugrohr (11) reiterförmig auf dem Blisterpackungs-Stab (5 bzw. 49) oder Käfig (34) sitzt.

41. Vorrichtung nach einem oder mehreren der vorhergehenden Ansprüche oder insbesondere danach, **dadurch gekennzeichnet, daß** das Saugrohr (11) in lösbarer Verbindung am Stab (5 bzw. 49) oder Käfig (34) sitzt und sich die Verbindung beim Einschieben des Stabes (5 bzw. 49) oder Käfigs (34) in den Träger (T) selbsttätig löst unter Übergabe des Saugrohres (11) an eine Führung des Trägers (T).

42. Vorrichtung nach einem oder mehreren der vorhergehenden Ansprüche oder insbesondere danach, **dadurch gekennzeichnet, daß** eine Flachseite des Saugrohres (11) Zapfen (54) aufweist, die reibschlüssig in Löcher (55) des Stabes (5), Kernstücks (49) oder Käfigs (34) eintreten.

43. Vorrichtung nach einem oder mehreren der vorhergehenden Ansprüche oder insbesondere danach, **dadurch gekennzeichnet, daß** das Lösen des Saugrohres (11) bei der Übergabe desselben an die Führung des Trägers (T) durch Schrägen (51) einlaufseitiger Hubnocken (61) einer Zahnreihe (62) bewirkt wird.

44. Vorrichtung nach einem oder mehreren der vorhergehenden Ansprüche oder insbesondere danach, **dadurch gekennzeichnet, daß** die Stirnseite eines zur Verrastung dienenden Käfigs (34) mit Benutzungssymbolen (47) ausgestattet ist.

45. Vorrichtung nach einem oder mehreren der vorhergehenden Ansprüche oder insbesondere danach, **dadurch gekennzeichnet, daß** die Stirnwand (40) des Käfigs (34) mit dem Winkel der Umwendbarkeit des Käfigs (34) entsprechend positionierten Originalitätsbrücken (77) ausgestattet ist, von denen die dem Deckel (4) jeweils zugekehrt liegende Originalitätsbrücke (77) durch eine Nase (80) des Deckels (4) bei der Schließbewegung desselben zerstörbar ist.

46. Vorrichtung nach einem oder mehreren der vorhergehenden Ansprüche oder insbesondere danach, **dadurch gekennzeichnet, daß** der kappenförmige Deckel (4) im Wege der Klapp-/Schiebebewegung in eine jenseits des rückwärtigen Gehäuseendes liegende Absenkstellung bewegbar ist.

47. Vorrichtung nach einem oder mehreren der vorhergehenden Ansprüche oder insbesondere danach, **dadurch gekennzeichnet, daß** der Deckel (4) einen quer abstehenden Ausleger (67) besitzt, der gelenkig und verschiebbar längs der oberen Gehäuseseite geführt ist und zusätzlich über einen Lenker (70) mit der unteren Gehäuseseite in Verbindung steht.

48. Vorrichtung nach einem oder mehreren der vorhergehenden Ansprüche oder insbesondere danach, **dadurch gekennzeichnet, daß** ein deckelseitiger Anlenkpunkt (72) des Lenkers (70) in einem Längsschlitz (73) des Deckels (4) verschieblich ist.

49. Vorrichtung nach einem oder mehreren der vorhergehenden Ansprüche oder insbesondere danach, **dadurch gekennzeichnet, daß** der Ausleger (67) paarig gestaltet ist und jeder Ausleger (67) mit einem Führungszapfen (68) in einem Führungsschlitz (69) des Gehäuses (3) zum vorderseitige Ende des Gehäuses (3) hin verschiebbar ist.

50. Vorrichtung nach einem oder mehreren der vorhergehenden Ansprüche oder insbesondere danach, **dadurch gekennzeichnet, daß** der Führungsschlitz (69) sich am rückwärtigen Gehäuseende in einen abwärtsgerichteten Abschnitt (75) fortsetzt derart, daß dieser Abschnitt(75) beim Verlagern des Deckels (4) jeweils eine Hub- bzw. Senkbewegung des Deckels (4) erzwingt derart, daß äessen Deckelrandkante (41') das abgeklappte Saugrohr (11) berührungslos überfährt.

51. Vorrichtung nach einem oder mehreren der vorhergehenden Ansprüche oder insbesondere danach, **dadurch gekennzeichnet, daß** der flächenförmige Träger (T) mit unterseitig daran ansitzendem Blister (2) in das Bodenteil des klappdeckelverschlossenen Gehäuses einbringbar ist, derart, daß Längsrippen (R) jeweils zwischen zwei Kavitätenreihen treten, welche Längsrippen im Scheitel deckend zu den Einzelschlitzen (83) des Trägers (T) und Blisters (2) Einstecköffnungen (83') für die freien Enden (82') der Laschen (82) besitzen.

52. Vorrichtung nach einem oder mehreren der vorhergehenden Ansprüche oder insbesondere danach, **dadurch gekennzeichnet, daß** der Träger (T) im Bereich seiner Ecken unterseitig Stifte (St) besitzt, die zur Fesselung des Blisters (2) am Träger (T) in Löchern (L) des Blisters eintreten.

## Claims

1. Device (V) for emptying powder-containing cavities (1), which are closed off by means of covering film or foil (9), by means of a suction tube (11), which can be applied manually and the front end of which, positioned and guided relative to the cavity (1), pierces the covering film or foil (9), leaving a clear cross section for air to flow into the cavity (1), **characterized in that** the front end of the suction tube (11) has, adjacent to and axially protruding with respect to cutting flanks (12) provided there, extensions (22/82) which are guided in a positively locking/rotationally fixed manner in slots (19 and/or 83, 55 or 25), adjacent to the cavities (1), of a carrier (T), into which carrier (T) the cavities (1) can be inserted as blister packs (2).

2. Device according to Claim 1 or in particular according thereto, **characterized by** the suction tube (11) being guided to the carrier (T) in such a manner that the suction tube (11) can move (only) in succession in two planes (E1, E2) which are in a relationship with respect to one another.

3. Device according to one or more of the preceding claims or in particular according thereto, **characterized by** at least two extensions (82) of the suction tube (11), which lie opposite one another with a distance between them and project beyond the cutting flanks in the axial direction of the suction tube (11).

4. Device according to one or more of the preceding claims or in particular according thereto, **characterized in that** the extensions (82) are formed as flat tabs (82).

5. Device according to one or more of the preceding claims or in particular according thereto, **characterized in that** the tabs (82) are displaceable in slots (19) and can then be introduced into individual slots (83) which lead from the base of the slots (19).

6. Device according to one or more of the preceding claims or in particular according thereto, **characterized in that** in one of the two planes (E1, E2) the suction tube (11) is displaceable both in a foldable manner and with linear guidance.

7. Device according to one or more of the preceding claims or in particular according thereto, **characterized by** suction tube positioning spaces (25/83) located beneath the slots (19).

8. Device according to one or more of the preceding claims or in particular according thereto, **characterized in that** the suction tube (11) is continuously displaceable in continuous slots (19) in the carrier (T), located on both sides of the holes (17).

9. Device according to one or more of the preceding claims or in particular according thereto, **characterized by** an additionally pivotably moveable association between the tabs (82) and the blister pack carrier (T) with the suction tube (11) not yet having been inserted.

10. Device according to one or more of the preceding claims or in particular according thereto, **characterized in that** the extensions (81) form articulated tabs through the intervention of carrier-side journals (89).

11. Device according to one or more of the preceding claims or in particular according thereto, **characterized in that** arcuate end edges of the tabs serve as guides during the positioning and introduction of the cutting edge of the suction tube (11) into the holes (17).

12. Device according to one or more of the preceding claims or in particular according thereto, **characterized in that** the tabs (22) which enter slots (19) in the blister pack carrier (T) and the suction tube (11), in this position, can be folded over the holes (17) so as to cover them.

13. Device according to one or more of the preceding claims or in particular according thereto, **characterized in that** the suction tube positioning spaces (25) are formed as branch passages which lead off from the slots (19), and the tabs (22) each have a cam (24) which can be introduced into the branch passages from the slot area.

14. Device according to one or more of the preceding claims or in particular according thereto, **characterized in that** the suction tube (11) has a plunger section (27), which can be introduced into the holes (17) and is disposed set back with respect to the piercing cutting flank, in such a manner that it is supplemented to form a linear guide for the suction tube (11) when the cams (24) enter the branch passages.

15. Device according to one or more of the preceding claims or in particular according thereto, **characterized in that** shoulders (28) of the suction tube (11), which adjoin the stub-like plunger section (27), in the final piercing position, come to rest on areas (29) which are lateral with respect to the holes (17) in the carrier (T).

16. Device according to one or more of the preceding claims or in particular according thereto, **characterized in that** the piercing cutting flank (cutting edge 12) extends to just above the base surface of the cavities (1) of the blister pack (5) and is adjoined by plunger blades (14) which extend less far.

17. Device according to one or more of the preceding claims or in particular according thereto, **characterized by** a holder, which can be folded by means of a lid, for storing the suction tube (11).

18. Device according to one or more of the preceding claims or in particular according thereto, **characterized by** a lid (4), which engages over the folded-down suction tube (11), of the carrier (T).

19. Device according to one or more of the preceding claims or in particular according thereto, **characterized in that** the narrow-side end wall (41) of the lid (4) secures the insertion position of the blister pack (2).

20. Device according to one or more of the preceding claims or in particular according thereto, **characterized in that** the carrier (T) which holds the blister pack (2) continues into a holder (87), into which the disengageable journals (89) enter, can overall, together with the blister pack (2), be placed into a housing (91) closed by a lid, in such a manner that drivers (91') which latch on the suction tube (11) fold the suction tube (11) upwards, pivoting about the journals (89), into a position in which it is ready to be gripped.

21. Device according to one or more of the preceding claims or in particular according thereto, **characterized by** a row of teeth (62), projecting in the manner of a comb, on each side wall of the housing (3), in the tooth spaces (53) of which the suction tube (11) is guided during the puncturing movement of the suction tube (11).

22. Device according to one or more of the preceding claims or in particular according thereto, **characterized in that** the vertically oriented puncturing movement of the suction tube (11) is guided via fins (52) of the suction tube (11), which run in the tooth spaces (53) of the housing (3), which tooth spaces (53) are arranged so as to match the pitch of the blister pack cavities (1).

23. Device according to one or more of the preceding claims or in particular according thereto, **characterized in that** the suction tube (11) latches (64, 65) in the lowered position.

24. Device according to one or more of the preceding claims or in particular according thereto, **characterized in that** the suction tube (11), on account of an articulated arrangement at its tabs (22), can be turned over about its transverse axis (56).

25. Device according to one or more of the preceding claims or in particular according thereto, **characterized in that** the tabs (22), which are arranged in pairs, are configured symmetrically with respect to turning and, in the folded-down position of the suction tube (11), are supported against the shoulders (28) of the latter.

26. Device according to one or more of the preceding claims or in particular according thereto, **characterized in that** the sheet-like carrier (T) for the blister pack (2) has individual insertion slots (84, 85) for the tabs (82) to enter.

27. Device according to one or more of the preceding claims or in particular according thereto, **characterized by** a blister pack (2) which, including covering film or foil (9), congruently with respect to the slots (83) in the carrier (T) and laterally adjacent to the cavities (1), has individual insertion slots (84, 85) for the extensions (82) to enter.

28. Device according to one or more of the preceding claims or in particular according thereto, **characterized in that** the length of the individual. insertion slots (84, 85) corresponds to the width of the tab-like extensions (82).

29. Device according to one or more of the preceding claims or in particular according thereto, **characterized in that** the carrier (T) is of bar-like configuration and, on a side face (16), has a rectilinear row of suction tube passage holes (17), which holes (17) are located congruently with respect to the blister pack cavities (1).

30. Device according to one or more of the preceding claims or in particular according thereto, **characterized in that** the holes (17) are connected to one another by narrower clear cross sections (18).

31. Device according to one or more of the preceding claims or in particular according thereto, **characterized by** a bar, which shapes the blister pack cavities (1), as core piece (49), the side faces (50) of which have the blister pack covering film or foil (9).

32. Device according to one or more of the preceding claims or in particular according thereto, **characterized in that** the blister pack bar (5) is polygonal in cross section, with internal cavities (1), and can be inserted into the carrier (T) in a plurality of positions in which it can be turned over about its longitudinal centre axis (x-x).

33. Device according to one or more of the preceding claims or in particular according thereto, **characterized in that** the blister pack bar (5) can be pushed into the carrier (T) in the longitudinal direction.

34. Device according to one or more of the preceding claims or in particular according thereto, **characterized in that** the blister pack bar (5) is square in cross section and has a row of cavities on all four wide sides.

35. Device according to one or more of the preceding claims or in particular according thereto, **characterized by** a folded blister pack hollow bar (5), which is surrounded by an insertion cage (34), which on all its side walls (36) forms openings (38) which coincide with the position of the cavities (1).

36. Device according to one or more of the preceding claims or 'in particular according thereto, **characterized in that** the carrier (T) has a core bar (30), which penetrates into the internal cross section (31) of the blister pack hollow bar (5) and of which at least the flank (32) which faces the suction tube (11) is configured as a supporting base for the underside (33) of the cavities.

37. Device according to one or more of the preceding claims or in particular according thereto, **characterized in that** all four flanks (32) of the core bar (30) are formed as a supporting base.

38. Device according to one or more of the preceding claims or in particular according thereto, **characterized in that** the blister pack hollow bar (5) latches in the insertion limit position.

39. Device according to one or more of the preceding claims or in particular according thereto, **characterized in that** the cavities (1) of all the side faces (50) are in each case located on a common transverse plane of the core piece (49).

40. Device according to one or more of the preceding claims or in particular according thereto, **characterized in that** the suction tube (11) is seated astraddle the blister pack bar (5 or 49) or cage (34).

41. Device according to one or more of the preceding claims or in particular according thereto, **characterized in that** the suction tube (11) is seated in a releasable connection on the bar (5 or 49) or cage (34), and the connection is automatically released when the bar (5 or 49) or cage (34) is pushed into the carrier (T), with the suction tube (11) being transferred to a guide of the carrier (T).

42. Device according to one or more of the preceding claims or in particular according thereto, **characterized in that** a flat side of the suction tube (11) has pegs (54) which, in a frictionally locking manner, enter holes (55) in the bar (5), core piece (49) or cage (34).

43. Device according to one or more of the preceding claims or in particular according thereto, **characterized in that** the release of the suction tube (11) when it is transferred to the guide of the carrier (T) is effected by bevels (51) of inlet-side lifting cams (61) of a row of teeth (62).

44. Device according to one or more of the preceding claims or in particular according thereto, **characterized in that** the end side of a cage (34) which is used for latching is equipped with utilization symbols (47).

45. Device according to one or more of the preceding claims or in particular according thereto, **characterized in that** the end wall (40) of the cage (34) is equipped with tamperproofing bridges (77) which are positioned in accordance with the angle through which the cage (34) can be turned over and of which the tamperproofing bridge (77) which in each case faces the lid (4) can be destroyed by a lug (80) of the lid (4) during the closing movement of the latter.

46. Device according to one or more of the preceding claims or in particular according thereto, **characterized in that** the cap-like lid (4), over the course of the folding/sliding movement, can be moved into a sunken position located beyond the rear end of the housing.

47. Device according to one or more of the preceding claims or in particular according thereto, **characterized in that** the lid (4) has a transversely protruding extension arm (67), which is guided pivotably and displaceably along the upper side of the housing and, in addition, is connected to the lower side of the housing via a link member (70).

48. Device according to one or more of the preceding claims or in particular according thereto, **characterized in that** a lid-side articulation point (72) for the link arm (70) is displaceable in a longitudinal slot (73) in the lid (4).

49. Device according to one or more of the preceding claims or in particular according thereto, **characterized in that** the extension arm (67) is of paired configuration, and each extension arm (67) can be displaced, by means of a guide peg (68) in a guide slot (69) in the housing (3), towards the front-side end of the housing (3).

50. Device according to one or more of the preceding claims or in particular according thereto, **characterized in that** the guide slot (69), at the rear end of the housing, continues into a downwardly directed section (75), in such a manner that this section (75), during the displacement of the lid (4), in each case imposes a lifting or lowering movement of the lid (4), in such a manner that its lid peripheral edge (41') moves over the folded-down suction tube (11) without touching it.

51. Device according to one or more of the preceding claims or in particular according thereto, **characterized in that** the sheet-like carrier (T), with blister (2) fitted to its lower side, can be introduced into the base part of the housing, which is closed off by a hinged lid, in such a manner that longitudinal ribs (R) in each case pass between two rows of cavities, which longitudinal ribs, at the vertex, have insertion openings (83'), which are congruent with respect to the individual slots (83) in the carrier (T) and in the blister (2), for the free ends (82') of the tabs (82).

52. Device according to one or more of the preceding claims or in particular according thereto, **characterized in that** the carrier (T), in the region of its corners, on the underside has pins (St) which, in order to retain the blister (2) on the carrier (T), move into holes (L) in the blister.

## Revendications

1. Dispositif (V) pour le vidage de cavités (1) contenant de la poudre et fermées par film de recouvrement (9) au moyen d'un tuyau d'aspiration (11) pouvant être placé à la main, dont l'extrémité avant est positionnée par rapport à la cavité (1) et passe à travers le film de recouvrement (9) de façon guidée en laissant une section transversale libre pour l'entrée d'air dans la cavité (1), **caractérisé en ce que** l'extrémité avant du tuyau d'aspiration (11) présente à proximité et débordant axialement par rapport à des flancs de coupe (12) prévus à cet endroit des prolongements (22/82), qui se guident par conjugaison de forme et de manière solidaire en rotation dans les fentes (19 et/ou 83, 55 ou 25), voisines dans les cavités (1) d'un support (T), support (T) dans lequel les cavités (1) peuvent être insérées sous la forme d'emballages transparents (2).

2. Dispositif selon la revendication 1 ou en particulier selon celle-ci, **caractérisé par** un guidage du tuyau d'aspiration (11) vers le support (T), en ce que le tuyau d'aspiration (11) est mobile (seulement) de façon successive dans deux plans situés l'un par rapport à l'autre (E1, E2).

3. Dispositif selon l'une quelconque ou plusieurs des revendications précédentes ou en particulier selon les revendications suivantes, **caractérisé par** au moins deux prolongements (82) du tuyau d'aspiration (11), faisant face à distance et dépassant des flancs de coupe dans la direction d'axe du tuyau d'aspiration (11).

4. Dispositif selon l'une quelconque ou plusieurs des revendications précédentes et en particulier selon les suivantes, **caractérisé en ce que** les prolongements (82) sont conçus comme des pattes (82) plates.

5. Dispositif selon l'une quelconque ou plusieurs des revendications précédentes ou en particulier selon les revendications suivantes, **caractérisé en ce que** les pattes (82) sont coulissantes dans des fentes (19) et peuvent entrer ensuite dans des fentes individuelles (83), qui sortent du fond des fentes (19).

6. Dispositif selon l'une quelconque ou plusieurs des revendications précédentes ou en particulier selon les revendications suivantes, **caractérisé en ce que**, dans l'un des deux plans (E1, E2), le tuyau d'aspiration (11) est coulissant aussi bien de façon repliable que guidé de façon linéaire.

7. Dispositif selon l'une quelconque ou plusieurs des revendications précédentes ou en particulier selon les revendications suivantes, **caractérisé par** des espaces libres de positionnement du tuyau d'aspiration (25/83) situés au-dessous des fentes (19).

8. Dispositif selon l'une quelconque ou plusieurs des revendications précédentes ou en particulier selon les revendications suivantes, **caractérisé en ce que** le tuyau d'aspiration (11) est coulissant de façon continue dans des fentes (19) du support (T) (continues, situées des deux côtés de trous (17).

9. Dispositif selon l'une quelconque ou plusieurs des revendications précédentes ou en particulier selon les revendications suivantes, **caractérisé par** une attribution en supplément mobile par pivotement des pattes (82) au support d'emballage transparent (T) avec un tuyau d'aspiration (11) non encore emboîté.

10. Dispositif selon l'une quelconque ou plusieurs des revendications précédentes ou en particulier selon les revendications suivantes, **caractérisé en ce que** les prolongements (81) forment des pattes articulées par l'entrée de tourillons d'axe (89) côté support.

11. Dispositif selon l'une quelconque ou plusieurs des revendications précédentes ou en particulier selon les revendications suivantes, **caractérisé en ce que** des arêtes frontales en forme d'arc des pattes servent de guides pour le positionnement et l'entrée de la lame du tuyau d'aspiration (11) dans les trous (17).

12. Dispositif selon l'une quelconque ou plusieurs des revendications précédentes ou en particulier selon les revendications suivantes, **caractérisé en ce que** les pattes (22) entrant dans des fentes (19) du support d'emballage transparent (T) et le tuyau d'aspiration (11) peuvent être repliés dans cette position par recouvrement sur les trous (17).

13. Dispositif selon l'une quelconque ou plusieurs des revendications précédentes ou en particulier selon les revendications suivantes, **caractérisé en ce que** les espaces libres de positionnement du tuyau d'aspiration (25) sont conçus comme des canaux de liaison partant des fentes (19) et les pattes (22) présentent chacune une came (24) et peuvent entrer à partir de la zone de fente dans les canaux de liaison.

14. Dispositif selon l'une quelconque ou plusieurs des revendications précédentes ou en particulier selon les revendications suivantes, **caractérisé en ce que** le tuyau d'aspiration (11) présente une partie de coulisseau (27) entrant dans les trous (17), qui est disposée de façon décalée en arrière par rapport aux flancs de coupe passant à travers et de telle sorte qu'elle se complète pour former un guide linéaire du tuyau d'aspiration (11) lorsque les cames (24) entrent dans les canaux de liaison.

15. Dispositif selon l'une quelconque ou plusieurs des revendications précédentes ou en particulier selon les revendications suivantes, **caractérisé en ce que** des épaulements (28), voisins de la partie de coulisseau (27) en forme de tubulure, du tuyau d'aspiration (11), sont posés, dans la position extrême de passage, sur des zones (29) sur le côté des trous (17) du support (T).

16. Dispositif selon l'une quelconque ou plusieurs des revendications précédentes ou en particulier selon les revendications suivantes, **caractérisé en ce que** le flanc de coupe passant à travers (lame 12) s'étend jusqu'à une distance juste au-dessus de la surface de fond des cavités (1) de l'emballage transparent (5) et est voisin de lames de coulisseau (14) allant moins loin.

17. Dispositif selon l'une quelconque ou plusieurs des revendications précédentes ou en particulier selon les revendications suivantes, **caractérisé par** une fixation, repliable au moyen d'un couvercle, pour la conservation du tuyau d'aspiration (11).

18. Dispositif selon l'une quelconque ou plusieurs des revendications précédentes ou en particulier selon les revendications suivantes, **caractérisé par** un couvercle (4) du support (T), recouvrant le tuyau d'aspiration (11) replié.

19. Dispositif selon l'une quelconque ou plusieurs des revendications précédentes ou en particulier selon les revendications suivantes, **caractérisé en ce que** le couvercle (4) bloque avec sa paroi avant (41) côté étroit la position d'introduction de l'emballage transparent (2).

20. Dispositif selon l'une quelconque ou plusieurs des revendications précédentes ou en particulier selon les revendications suivantes, **caractérisé en ce que** le support (T) recevant l'emballage transparent (2) se prolonge dans une fixation (87) dans laquelle entrent les tourillons d'axe (89) pouvant être désengagés, peut être placé globalement avec l'emballage transparent (2) dans un boîtier (91) fermé par un couvercle de telle sorte que les entraîneurs (91') s'engageant sur le tuyau d'aspiration (11) relèvent le tuyau d'aspiration (11) par pivotement autour des tourillons d'axe (89) dans une position prête pour la préhension.

21. Dispositif selon l'une quelconque ou plusieurs des revendications précédentes ou en particulier selon les revendications suivantes, **caractérisé par** une rangée de dents (62) dépassant à la façon d'un peigne sur chaque paroi latérale du boîtier (3), dans les entredents (53) duquel le tuyau d'aspiration (11) se guide lors du mouvement de percée du tuyau d'aspiration (11).

22. Dispositif selon l'une quelconque ou plusieurs des revendications précédentes ou en particulier selon les revendications suivantes, **caractérisé en ce que** le mouvement de percée orienté verticalement du tuyau d'aspiration (11) est guidé par des nervures (52) du tuyau d'aspiration (11), qui vont dans les entredents (53) du boîtier (3), lesquels entredents (53) sont disposés en fonction de la division des cavités d'emballage transparent (1).

23. Dispositif selon l'une quelconque ou plusieurs des revendications précédentes ou en particulier selon les revendications suivantes, **caractérisé en ce que** le tuyau d'aspiration (11) est encliqueté (64, 65) dans la position emboîtée inférieure.

24. Dispositif selon l'une quelconque ou plusieurs des revendications précédentes ou en particulier selon les revendications suivantes, **caractérisé en ce que** le tuyau d'aspiration (11) peut être retourné autour de son axe transversal (56) en raison de la disposition articulée sur ses pattes (22).

25. Dispositif selon l'une quelconque ou plusieurs des revendications précédentes ou en particulier selon les revendications suivantes, **caractérisé en ce que** les pattes (22) disposées par paires sont conçues de façon symétrique en retournement et s'appuient sur les épaulements (28) du tuyau d'aspiration (11) lorsque celui-ci est dans la position repliée.

26. Dispositif selon l'une quelconque ou plusieurs des revendications précédentes ou en particulier selon les revendications suivantes, **caractérisé en ce que** le support (T) en forme de surface pour l'emballage transparent (2) présente des fentes d'introduction individuelles (84, 85) pour l'entrée des pattes (82).

27. Dispositif selon l'une quelconque ou plusieurs des revendications précédentes ou en particulier selon les revendications suivantes, **caractérisé par** un emballage transparent (2) qui présente avec le film de recouvrement (9), de façon couvrante par rapport aux fentes (83) dans le support (T) et voisines sur le côté des cavités (1), des fentes d'emboîtement individuelles (84, 85) pour l'entrée des prolongements (82).

28. Dispositif selon l'une quelconque ou plusieurs des revendications précédentes ou en particulier selon les revendications suivantes, **caractérisé en ce que** la longueur des fentes d'emboîtement individuelles (84, 85) correspond à la largeur des prolongements (82) en forme de pattes.

29. Dispositif selon l'une quelconque ou plusieurs des revendications précédentes ou en particulier selon les revendications suivantes, **caractérisé en ce que** le support (T) est conçu en forme de barre et présente sur une surface latérale (16) une rangée rectiligne de trous de passage du tuyau d'aspiration (17), lesquels trous (17) chevauchent les cavités d'emballage transparent (1).

30. Dispositif selon l'une quelconque ou plusieurs des revendications précédentes ou en particulier selon les revendications suivantes, **caractérisé en ce que** les trous (17) sont reliés entre eux par des sections transversales (18) libres assez étroites.

31. Dispositif selon l'une quelconque ou plusieurs des revendications précédentes ou en particulier selon les revendications suivantes, **caractérisé par** une barre formant les cavités d'emballage transparent (1) en tant que pièce maîtresse (49), dont les faces latérales (50) présentent le film de recouvrement de l'emballage transparent (9).

32. Dispositif selon l'une quelconque ou plusieurs des revendications précédentes ou en particulier selon les revendications suivantes, **caractérisé en ce que** la barre d'emballage transparent (5) présente une section transversale à plusieurs côtés avec des cavités (1) intérieures et peut être insérée dans le support (T) dans plusieurs positions pouvant tourner autour de son axe médian longitudinal (x-x).

33. Dispositif selon l'une quelconque ou plusieurs des revendications précédentes ou en particulier selon les revendications suivantes, **caractérisé en ce que** la barre d'emballage transparent (5) peut être introduite dans le sens longitudinal dans le support (T).

34. Dispositif selon l'une quelconque ou plusieurs des revendications précédentes ou en particulier selon les revendications suivantes, **caractérisé en ce que** la barre d'emballage transparent (5) présente une section transversale carrée et a une rangée de cavités sur chacun des quatre côtés larges.

35. Dispositif selon l'une quelconque ou plusieurs des revendications précédentes ou en particulier selon les revendications suivantes, **caractérisé par** une barre creuse d'emballage transparent (5) pliée, qui est entourée par une cage d'introduction (34), laquelle présente sur l'ensemble de ses parois latérales (37) des ouvertures (38) coïncidant avec la position des cavités (1).

36. Dispositif selon l'une quelconque ou plusieurs des revendications précédentes ou en particulier selon les revendications suivantes, **caractérisé en ce que** le support (T) présente une barre principale (30) plongeant dans la section transversale intérieure (31) de la barre creuse d'emballage transparent (5), dont au moins le flanc (32) tourné vers le tuyau d'aspiration (11) est conçu comme fond de support pour le côté inférieur des cavités (33).

37. Dispositif selon l'une quelconque ou plusieurs des revendications précédentes ou en particulier selon les revendications suivantes, **caractérisé en ce que** tous les quatre flancs (32) de la barre principale (30) sont conçus comme fond de support.

38. Dispositif selon l'une quelconque ou plusieurs des revendications précédentes ou en particulier selon les revendications suivantes, **caractérisé en ce que** la barre creuse d'emballage transparent (5) s'enclenche dans la position extrême d'introduction.

39. Dispositif selon l'une quelconque ou plusieurs des revendications précédentes ou en particulier selon les revendications suivantes, **caractérisé en ce que** les cavités (1) de toutes les faces latérales (50) reposent respectivement sur un plan transversal commun de la pièce principale (49).

40. Dispositif selon l'une quelconque ou plusieurs des revendications précédentes ou en particulier selon les revendications suivantes, **caractérisé en ce que** le tuyau d'aspiration (11) repose en forme de cavalier sur la barre d'emballage transparent (5 ou 49) ou sur la cage (34).

41. Dispositif selon l'une quelconque ou plusieurs des revendications précédentes ou en particulier selon les revendications suivantes, **caractérisé en ce que** le tuyau d'aspiration (11) repose dans une liaison amovible sur la barre (5 ou 49) ou la cage (34) et la liaison se détache toute seule lors de l'introduction de la barre (5 ou 49) ou de la cage (34) dans le support (T) avec le transfert du tuyau d'aspiration (11) à un guide du support (T).

42. Dispositif selon l'une quelconque ou plusieurs des revendications précédentes ou en particulier selon les revendications suivantes, **caractérisé en ce qu'**un côté plat du tuyau d'aspiration (11) présente des tenons (54) qui entrent par liaison à friction dans des trous (55) de la barre (5), de la pièce principale (49) ou de la cage (34).

43. Dispositif selon l'une quelconque ou plusieurs des revendications précédentes ou en particulier selon les revendications suivantes, **caractérisé en ce que** le détachement du tuyau d'aspiration (11) est entraîné, lors du transfert de ce tuyau au guide du support (T), par des chanfreins (51) de cames de levage (61) côté entrée d'une rangée de dents (62).

44. Dispositif selon l'une quelconque ou plusieurs des revendications précédentes ou en particulier selon les revendications suivantes, **caractérisé en ce que** le côté avant d'une cage (34) servant à l'encliquetage est équipé de symboles d'utilisation (47).

45. Dispositif selon l'une quelconque ou plusieurs des revendications précédentes ou en particulier selon les revendications suivantes, **caractérisé en ce que** la paroi frontale (40) de la cage (34) est équipée de ponts originaux (77) positionnés en fonction de l'angle de retournement possible de la cage (34), dont le pont original (77) respectivement tourné vers le couvercle (4) peut être détruit par un ergot (80) du couvercle (4) lors du mouvement de fermeture de celui-ci.

46. Dispositif selon l'une quelconque ou plusieurs des revendications précédentes ou en particulier selon les revendications suivantes, **caractérisé en ce que** le couvercle (4) en forme de capuchon peut être déplacé par le biais du mouvement de repliage/coulissement dans une position d'abaissement située au-delà de l'extrémité de boîtier côté arrière.

47. Dispositif selon l'une quelconque ou plusieurs des revendications précédentes ou en particulier selon les revendications suivantes, **caractérisé en ce que** le couvercle (4) présente une flèche (67) débordant transversalement, qui est guidée de façon articulée et coulissante le long du côté supérieur du boîtier et est en liaison en supplément au moyen d'un bras oscillant (70) avec le côté inférieur du boîtier.

48. Dispositif selon l'une quelconque ou plusieurs des revendications précédentes ou en particulier selon les revendications suivantes, **caractérisé en ce qu'**un point d'articulation (72) côté couvercle du bras oscillant (70) peut coulisser dans une fente longitudinale (73) du couvercle (4).

49. Dispositif selon l'une quelconque ou plusieurs des revendications précédentes ou en particulier selon les revendications suivantes, **caractérisé en ce que** la flèche (67) est logée par paires et chaque flèche (67) peut coulisser avec un tenon de guidage (68) dans une fente de guidage (69) du boîtier (3) en direction de l'extrémité avant du boîtier (3).

50. Dispositif selon l'une quelconque ou plusieurs des revendications précédentes ou en particulier selon les revendications suivantes, **caractérisé en ce que** la fente de guidage (69) se prolonge sur l'extrémité arrière du boîtier dans une partie (75) orientée vers le bas de telle sorte que cette partie (75) force effectivement un mouvement de levage ou d'abaissement du couvercle (4) lors du déplacement du couvercle (4) de telle sorte que son arête de bordure de couvercle (41') passe au-dessus du tuyau d'aspiration (11) replié sans contact.

51. Dispositif selon l'une quelconque ou plusieurs des revendications précédentes ou en particulier selon les revendications suivantes, **caractérisé en ce que** le support (T) en forme de surface peut être introduit avec l'emballage (2) logé sur le côté inférieur du support dans la partie de fond du boîtier fermé par un couvercle repliable, de telle sorte que des nervures longitudinales (R) arrivent respectivement entre deux rangées de cavités, lesquelles nervures longitudinales présentent au sommet et en recouvrement avec les fentes individuelles (83) du support (T) et de l'emballage (2) des orifices d'emboîtement (83') pour les extrémités libres (82') des pattes (82).

52. Dispositif selon l'une quelconque ou plusieurs des revendications précédentes ou en particulier selon les revendications suivantes, **caractérisé en ce que** le support (T) présente dans la zone de ses angles côté inférieur des broches (St) qui entrent dans des trous (L) de l'emballage pour le blocage de l'emballage (2) sur le support (T).
